# EUROPEAN PATENT APPLICATION

(11) **EP 4 434 341 A1**
(43) Date of publication of application: **25.09.2024**
(21) Application number: 22892154.0
(22) Date of filing: 15.11.2022
(51) Int. Cl.: A01N 1/02, A61K 45/00, C12Q 1/48

(54) **NEW METHOD FOR IMPROVING QUALITY OF PLATELETS**

(30) Priority: 15.11.2021 CN 202111346275
(71) Applicant: SHANGHAI SYNVIDA BIOTECHNOLOGY CO. LTD., Shanghai 201303 (CN)
(72) Inventor: LIU, Junling, Shanghai 201203 (CN); FAN, Xuemei, Shanghai 201203 (CN)
(74) Representative: Delbarba, Andrea
(86) International application number: PCT/CN2022/131812
(87) International publication number: WO 2023/083361

(57) **Abstract**

Disclosed is a new method for improving the quality of platelets. It is disclosed for the first time that the molecular of carnitine palmitoyl transferase II (CPT2) of the platelet mitochondrial inner membrane is critical to the survival, preparation and storage of platelets, the reduction of CPT2 protein expression and/or activity seriously affects the quality of platelets, and the normal or high expression of the protein improves the quality of platelets. By means of directly and indirectly influencing or modifying CPT2 protein, the effect of CPT2 on platelets is verified at multiple angles, and a pathway and endogenous or exogenous molecules having a key effect of maintaining activity of platelets are disclosed. Therefore, the CPT2 itself or an up-regulator thereof can be used for maintaining the activity of platelets, and maintaining or improving the storage quality of platelets, and can also be used for screening substances capable of maintaining the activity of platelets or improving the storage quality thereof on the basis of the CPT2 or the signal pathway involving same and using same as a target.

## Description

This application claims priority to Chinese application No. 202111346275.7 filed on November 15, 2021.

### FIELD OF THE INVENTION

The present disclosure relates to the field of biomedicine. More specifically, the present disclosure refers to a new method for improving quality of platelets.

### BACKGROUND

Platelets are unique anucleate blood cells of mammals, comprising mitochondria, granules, lysosomes and other subcellular structures and evolving from directly proliferated hematopoietic stem cells. The lifespan of each platelet is very short. The lifespan of platelets in blood circulation in vivo is about 7-14 days. When a life cycle of platelets completed, spleen and liver are responsible for removing the aged platelets.

The platelet count in human peripheral blood under normal conditions is about 1 to 3×10¹¹ platelets. It is very important to maintain a certain number of platelets in peripheral blood. Thrombocytopenia may be induced by systemic anticoagulation therapy, traumatic surgery, fatal bleeding, chemotherapy and radiotherapy in patients. A decrease of platelets in circulating blood may resulted from infections, immune thrombocytopenia, or other hemorrhagic diseases in individuals. A platelet count under 2×10¹⁰ may cause severe life-threatening bleeding. At this time, the patients must be transfused with platelets to prevent possible uncontrolled bleeding or to correct the bleeding time. However, due to individual differences in clinical, platelets exhibit significant variations in lifespan and quality both in vivo and in vitro. Additionally, platelet transfusion is a crucial clinical treatment method, but the demand for apheresis platelets often exceeds the available supply.

Currently, platelets used clinically are mainly obtained through single-component blood donation, where platelets are selectively collected using apheresis blood donation instruments. The instrument withdraws blood from the donor and separates platelets from other blood cells internally. Subsequently, the concentrated platelet-rich plasma is collected into a storage bag, while the other components of the blood are returned to the donor's body.

However, platelets are highly sensitive to the environment. One storage method involves preparing cryopreserved platelets by adding glycerol and DMSO and storing at -80°C. When needed, these platelets are thawed. After thawing, irreversible fibrinogen precipitation may occur in the platelet products, indicating platelet activation. Cryopreserved platelets are mainly used for emergency platelet transfusions when immediate hemostasis is required. Studies have shown that cryopreservation can cause irreversible changes in platelet membranes, leading to rapid clearance by hepatic and splenic macrophages after transfusion. Therefore, the storage temperature of platelets is crucial for their survival in the circulatory system after transfusion. These limitations restrict the widespread clinical use of cryopreserved platelets. Additionally, apheresis platelets have a shelf life of only 5 days. Platelet aging and metabolism can affect their senescence, apoptosis, and pathological death. Moreover, prior to transfusion, platelets must undergo testing of blood typing, pathogen and other factors, resulting in a very short clinical usage window and low platelet viability upon transfusion. Therefore, there is an urgent need in this field for a method that can improve platelet lifespan and quality.

### SUMMARY

The aim of the present disclosure is to provide a novel target that regulates platelet viability or quality, that is carnitine palmitoyltransferase II (CPT2) molecule; the aim of the present disclosure is also to provide regulatory molecules that directly or indirectly acting on this target, and the uses thereof.

In the first aspect, the present disclosure provides a use of carnitine palmitoyltransferase II or an up-regulator in preparation of a composition, wherein the composition has one or more of the following functions: (1) maintaining or improving the activity of platelets, (2) maintaining or improving storage quality of platelets, (3) reducing platelet apoptosis, (4) prolonging platelet lifespan and/or (5) increasing platelet counts.

In one or more embodiments, the CPT2 dysfunction leads to fatty acid metabolism disorder, affecting the activity, quality, quantity and/or lifespan of platelets. An up-regulator thereof alleviates or improves this situation.

In one or more embodiments, the fatty acid metabolism disorder comprises: accumulation of long-chain fatty acylcarnitines (C16:0, C18:0) with the increase of time.

In one or more embodiments, the fatty acid metabolism disorder comprises: increased concentration of fatty acylcarnitine in platelet plasma.

In one or more embodiments, the platelets are ex vivo platelets or in vivo platelets.

In one or more embodiments, the platelets are platelets of mammalian origin, such as human platelets, mouse platelets, monkey platelets or artificial platelets.

In one or more embodiments, the platelets are platelet-rich plasma (PRP).

In one or more embodiments, the platelets comprise platelets stored at room temperature and/or stored with refrigeration.

In one or more embodiments, the reduction of platelet apoptosis refers to a decrease of at least 5%, 10%, or 20% in the percentage A1 of apoptotic platelets in platelet samples after administration of the composition compared to the percentage A0 of apoptotic platelets in platelet samples without administration of the composition.

In one or more embodiments, the prolonging of platelet lifespan refers to a prolonging T1/T0 ratio of at least 6, 12, 24, 36, 48, or 72 hours, wherein the T1/T0 ratio is the platelet survival time T1 after administration of the composition compared to the platelet survival time T0 without administration of the composition.

In one or more embodiments, the increase in platelet counts refers to a P1/P0 ratio of at least 1.5, preferably at least 2, 3, 4, 5, 6, 7, 8, 9, 10, 20, 30, 50, or 100, wherein the P1/P0 ratio is the platelet quantity P1 after administration of the composition compared to the platelet quantity P0 without administration of the composition.

In one or more embodiments, the up-regulator of carnitine palmitoyltransferase II comprises (but are not limited to) one or more selected from the following group: (a) a substance capable of reducing acetylation of carnitine palmitoyltransferase II; (b) a substance capable of increasing activity of carnitine palmitoyltransferase II; (c) a substance capable of improving expression, stability or effective time of carnitine palmitoyltransferase II.

In one or more embodiments, the up-regulator of carnitine palmitoyltransferase II comprises (but are not limited to) one or more selected from the following group:
an agent targeting acetylation site of carnitine palmitoyltransferase II to reduce or abolish acetylation of the site; preferably, the substance that reduces acetylation of carnitine palmitoyltransferase II is a protein acetylation inhibitor; preferably, the protein acetylation inhibitor comprises a down-regulator that reduces acetyltransferase gene or protein expression and/or activity; preferably, the protein acetylation inhibitor is a mitochondrial protein acetylation inhibitor; preferably, the protein acetylation inhibitor is specific to CPT2; preferably, the acetylation site is lysine 79 of carnitine palmitoyltransferase II; preferably, the agent comprises (but are not limited to): an agent for site-directed mutagenesis or site-directed modification; preferably, the down-regulator that reduces acetyltransferase gene or protein expression and/or activity comprises down-regulators of gene editing, gene knockout, siRNA, microRNA, or small molecules; preferably, the protein acetylation inhibitor is SIRT3 or an activator thereof;
CPT1 inhibitors; preferably, the CPT1 inhibitors comprise CPT1 inhibitors to itself, upstream AMPK inhibitors (AMPKi), ACC agonists; preferably, the CPT1 inhibitors comprise (but are not limited to): Malonyl-CoA (malonyl coenzyme A), Etomoxir (Etomoxir), Perhexiline maleate (perhexiline maleate), Dorsomorphin 2HCl;
protein acetylation inhibitors; preferably comprising (but are not limited to): lysine acetyltransferase inhibitors, mitochondrial protein acetylation inhibitors, or activators or expression up-regulators thereof; preferably, the lysine acetyltransferase inhibitors comprise (but are not limited to): tip60 inhibitors or p300 inhibitors; preferably, the mitochondrial protein acetylation inhibitors comprise (but are not limited to): SIRT3; preferably, activators of SIRT3 comprise (but are not limited to) (direct activator or indirect activator): agents that increase concentration of NAD[+] precursors or agents that increase NAMPT enzyme activity; preferably, the agents that increases concentration of NAD[+] precursors comprise (but are not limited to): nicotinamide mononucleotide (NMN), nicotinamide (NAM), nicotinamide riboside (NR); preferably, the agents that increase NAMPT enzyme activity comprise (but are not limited to): P7C3, SBI-797812;
AMPK inhibitors; preferably comprising (but are not limited to): Dorsomorphin 2HCl;
antioxidants; preferably comprising (but are not limited to): N-acetylcysteine (NAC), Mesna or glutathione;
an expression construct (including expression vectors) for recombinantly expressing carnitine palmitoyltransferase II;
an up-regulator that promotes the driving ability of carnitine palmitoyltransferase II gene promoter; or
a down-regulator of carnitine palmitoyltransferase II gene-specific microRNA.

In one or more embodiments, the expression construct (expression vectors) comprise: viral vectors, non-viral vectors; preferably, the expression vectors comprise: adeno-associated virus vectors, lentiviral vectors, adenoviral vectors.

In one or more embodiments, the acetyltransferases comprise (but are not limited to): tip60 inhibitors or p300 inhibitors.

In another aspect, the present disclosure provides a method for storing platelets in vitro and/or improving the quality of platelets stored in vitro, wherein the method comprises (but are not limited to) steps selected from the following group during platelet storage: (a) decreasing acetylation of carnitine palmitoyltransferase II (CPT2); (b) enhancing activity of carnitine palmitoyltransferase II; (c) increasing the expression, stability, or effective time of carnitine palmitoyltransferase II; or (d) adding active carnitine palmitoyltransferase II (non-acetylating enzyme).

In one or more embodiments, the tip60 inhibitor is used in an amount of 0.1-100 umol/L (e.g., 0.5, 1, 2, 5, 10, 20, 30, 50, 60, 80 umol/L).

In one or more embodiments, the p300 inhibitor is used in an amount of 0.1-100 umol/L (e.g., 0.5, 1, 2, 5, 10, 20, 30, 50, 60, 80 umol/L).

In one or more embodiments, the NMN is used in an amount (final concentration) of 0.05-50 mM (e.g., 0.08, 0.1, 0.2, 0.5, 0.8, 1, 1.5, 2, 3, 5 or 10mM), preferably 0.1-10mM, more preferably 0.2-5mM.

In one or more embodiments, the nicotinamide is used in an amount (final concentration) of 0.05-10 mM (e.g., 0.08, 0.1, 0.2, 0.5, 0.8, 1, 1.5, 2, 3, 5 or 10mM), preferably 0.1-10mM, more preferably 0.2-5mM.

In one or more embodiments, the nicotinamide riboside is used in an amount (final concentration) of 0.05-50 mM (e.g., 0.08, 0.1, 0.2, 0.5, 0.8, 1, 1.5, 2, 3, 5 or 10mM), preferably 0.1-10mM, more preferably 0.2-5mM.

In one or more embodiments, the P7C3 is used in an amount (final concentration) of 0.025-50uM (e.g., 0.04, 0.05, 0.2, 0.4, 0.5, 1, 2.5, 4, 5, 8, 10, 15, 25 or 40uM), preferably 0.05-30uM, more preferably 0.25-15uM.

In one or more embodiments, the SBI-797812 is used in an amount (final concentration) of 0.1-200 uM (e.g., 0.15, 0.2, 0.8, 1.5, 2, 4, 10, 15, 20, 30, 40, 60, 100 or 150uM), preferably 0.2-120uM, more preferably 1-60uM.

In one or more embodiments, the Dorsomorphin 2HCl is used in an amount of 0.05-80 uM (e.g., 0.08, 0.1, 0.15, 0.2, 0.3, 0.5, 0.5, 1, 2, 5, 10, 15, 20, 30, 40, 60 or 70uM), preferably 0.1-50uM, more preferably 0.5-20uM.

In one or more embodiments, the N-acetylcysteine is used in an amount of 0.05-500mM (e.g.,0.08, 0.1, 0.2, 0.5, 0.8, 1, 1.5, 2, 3, 5, 10, 20, 50, 80, 100, 200mM).

In one or more embodiments, the Mesna is used in an amount of 1-1000uM (e.g., 1.5, 2, 4, 10, 15, 20, 30, 40, 60, 100, 150, 200, 400, 600, 800uM).

In one or more embodiments, the glutathione is used in an amount of 10-2000uM (e.g., 15, 20, 30, 40, 60, 100, 150, 200, 400, 600, 800, 1000, 1200, 1500, 1800uM).

In one or more embodiments, the "amount" comprises "adding amount", preferably comprises: the final concentration of the corresponding additive in the composition (including the composition hereinafter), the final concentration of the corresponding additive in platelets or substances comprising platelets.

In one or more embodiments, the "substances comprising platelets" comprise naturally collected (for example freshly collected) blood/plasma comprising platelets, blood products enriched with platelets, processed blood products.

In another aspect, the present disclosure provides a method for maintaining or improving activities of platelets, comprising (but are not limited to) administering the following treatments to the subjects in need of improving platelet activities: (a) decreasing acetylation of carnitine palmitoyltransferase II (CPT2); (b) enhancing activity of carnitine palmitoyltransferase II; (c) increasing the expression, stability, or effective time of carnitine palmitoyltransferase II; or (d) adding active carnitine palmitoyltransferase II (non-acetylating enzyme).

In another aspect, the present disclosure provides a composition, wherein the composition has one or more functions selected from the following group: (1) maintaining or improving the activity of platelets, (2) maintaining or improving storage quality of platelets, (3) reducing platelet apoptosis, (4) prolonging platelet lifespan and/or (5) increasing platelet counts; wherein the composition comprises an up-regulator of carnitine palmitoyltransferase II.

In one or more embodiments, the composition comprises: a protein acetylation inhibitor, an antioxidant and a CPT1 inhibitor.

In one or more embodiments, the protein acetylation inhibitor is NMN, the antioxidant is NAC, the CPT1 inhibitor is AMPKi.

In one or more embodiments, the molar ratio of NMN, NAC and AMPKi is (50-500):(500-5000):1; more preferably (80-300):(800-3000):1 (for example, 90:900:1, 100:1000:1, 110:1110:1, 120:1200:1, 125:1250:1, 140:1400:1, 150:1500:1, 180:1800: 1, 200: 2000: 1, 250: 2500: 1, etc.).

In one or more embodiments, when NMN, NAC and AMPKi are used in combination, the concentration of the NAD+ precursor NMN added in platelets or substances comprising platelets (such as plasma, platelet-rich plasma, etc.) is 0.1-5000uM (such as 0.2, 0.5, 1, 2, 5, 10, 20, 50, 100, 200, 500, 800, 1000, 1500, 2000, 3000, 4000uM).

In one or more embodiments, when NMN, NAC and AMPKi are used in combination, the concentration of the N-acetylcysteine in platelets or substances comprising platelets is 1-50000uM (such as 2, 5, 10, 20, 50, 100, 200, 500, 1000, 2000, 5000, 8000, 10000, 15000, 20000, 30000, 40000uM).

In one or more embodiments, when NMN, NAC and AMPKi are used in combination, the concentration of the AMPKi in platelets or substances comprising platelets is 0.8-40000nM (such as.6, 4, 8, 16, 40, 80, 160, 400, 800, 1600, 4000, 6400, 8000, 12000, 16000, 24000, 32000 nM).

In another aspect, the present disclosure provides a drug kit, comprising the composition.

In another aspect, the present disclosure provides a use of the composition or the drug kit for (1) maintaining or improving the activity of platelets, (2) maintaining or improving storage quality of platelets, (3) reducing platelet apoptosis, (4) prolonging platelet lifespan and/or (5) increasing platelet counts.

In another aspect, the present disclosure provides a use of carnitine palmitoyltransferase II for screening drugs (i.e. as drug screening targets), wherein the drug has a function selected from the following group: (1) maintaining or improving the activity of platelets, (2) maintaining or improving storage quality of platelets, (3) reducing platelet apoptosis, (4) prolonging platelet lifespan and/or (5) increasing platelet counts.

In another aspect, the present disclosure provides a method for screening drugs (or potential drugs) able of maintaining or improving the activity of platelets, or maintaining or improving storage quality of platelets, wherein the method comprises:
(1) treating an expression system by a candidate substance, wherein the system expressing carnitine palmitoyltransferase II; and
(2) detecting the carnitine palmitoyltransferase II in the system, if the candidate substance has a function selected from the following group, it indicates that the candidate substance is the desired (interesting) drug;
   (i) reducing or abolishing the acetylationn at the acetylation site of carnitine palmitoyltransferase II; preferably, the acetylation site is lysine 79 of carnitine palmitoyltransferase II;
   (ii) improving expressions of carnitine palmitoyltransferase II; or
   (iii) improving activities of carnitine palmitoyltransferase II.

In one or more embodiments, the step (1) comprises: in the testing group, adding candidate substances to the expression system; and/or, the step (2) comprises: detecting the system to observe the expression or activity of carnitine palmitoyltransferase II and compared to the control group, wherein the control group is an expression system without adding the candidate substances; if any one of the functions (i)-(iii) of carnitine palmitoyltransferase II is elevated statistically (such as elevated by more than 20%, preferably more than 50%; more preferably more than 80%) by the candidate substance, then it indicates that the candidate substance is the desired (interesting) drug or compound.

In one or more embodiments, the system is selected from: cell system (or cell culture system), subcellular system (or subcellular culture system), solution system, tissue system, organ system or animal system.

In one or more embodiments, the candidate substance comprises (but is not limited to): overexpressed molecules for example constructs active promoters, chemical small molecules, interacting molecules targeting carnitine palmitoyltransferase II, fragments or variants thereof, encoding genes thereof or upstream or downstream molecules or molecules in signaling pathway.

In one or more embodiments, the method also comprises: further cell experiments and/or animal experiments on the obtained drug or compound (potential drug or compound), so as to further select and determine compositions useful for maintaining or improving the activity of platelets, maintaining or improving storage quality of platelets.

In another aspect, the present disclosure provides a genetically engineered platelet or a precursor thereof, wherein there is an exogenous carnitine palmitoyltransferase II or a up-regulator thereof converted in the platelet or the precursor; preferably, the up-regulator is a biomolecule, such as a polynucleotide, a construct comprising a polynucleotide or a protein. Preferably, the up-regulator of carnitine palmitoyltransferase II is an agent targeting acetylation site of carnitine palmitoyltransferase II to reduce or abolish acetylation of the site; more preferably, the agent targeting the acetylation site of carnitine palmitoyltransferase II, lysine 79, making it mutated.

In one or more embodiments, the precursor is a megakaryocyte and/or a fibroblast.

In another aspect, the present disclosure provides a composition/culture system (culture) comprising the genetically engineered platelet or a precursor thereof.

In another aspect, the present disclosure provides a drug kit comprising the genetically engineered platelet or a precursor thereof, or the composition/culture system (culture).

Other aspects of the present disclosure will be apparent to those skilled in the art based on the disclosure herein.

### BRIEF DESCRIPTION OF DRAWINGS

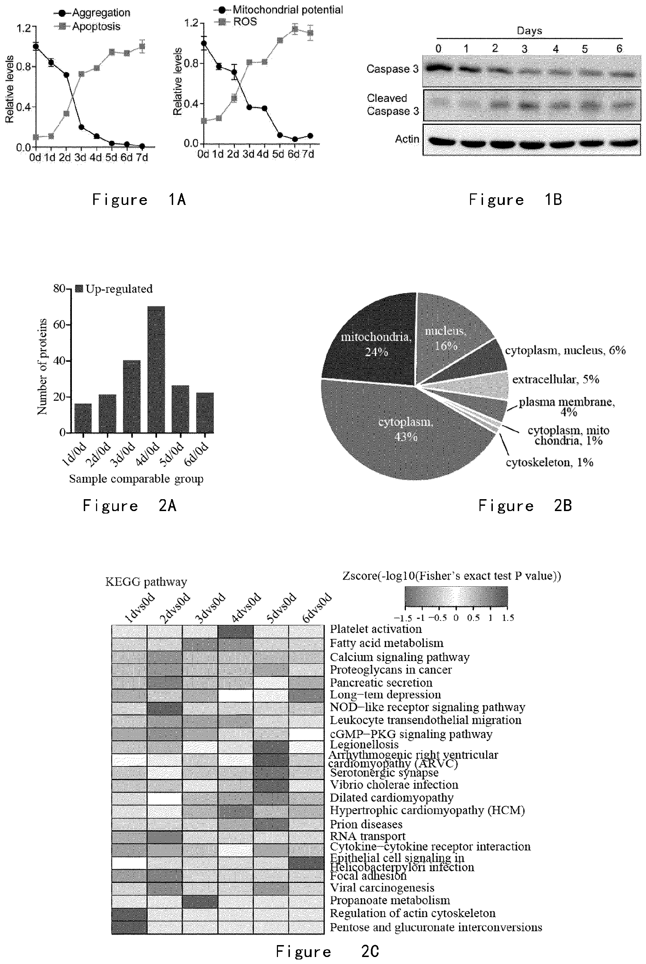

Figure 1A. The analysis of stored platelets for different days showed that with the increase of storage time, the function of platelets declines and the apoptosis ratio of platelets gradually increases.
Figure 1B. The activation of Caspase3 was real-time detected by using Western blot analysis. Caspase3 activation increases with the increase of days of platelet storage. The molecular weight of Caspase3 is 35kd and the molecular weight of the shear-activated Caspase3 is 17kd.
Figure 2A. Through quantitative proteomic analysis of acetylation modifications on platelets stored for different days, it was found that the protein modification in platelets changed during storage, with a significantly changed acetylation level of proteins within platelets on the third or fourth day of storage.
Figure 2B. Statistical analyses of the subcellular location of differentially expressed proteins, wherein up to a quarter of protein species are originated from mitochondria.
Figure 2C. A cluster analysis of KEGG pathway enrichment was carried out based on the experimental results, with a heat map visualized. It was found that the proteins with altered acetylation levels were mainly distributed in the cytoplasm and mitochondria. Most of the proteins with altered acetylation levels were involved in biochemical processes such as platelet activation and metabolism. Among them, many metabolism-related enzymes have been found to be acetylated, such as glutamate oxaloacetate transaminase (GOT2)/ propionyl-CoA carboxylase (PCCA)/ dihydrolipoamide dehydrogenase (DLD)/acyl-CoA dehydrogenase medium chain (ACADM)/ carnitine palmitoyltransferase 2 (CPT2)/ acetyl-CoA acetyltransferase 1 (ACAT1)/mitochondrial trifunctional protein (HADHA/HADHB)/very long-chain acyl-CoA dehydrogenase (ACADVL) and so on.

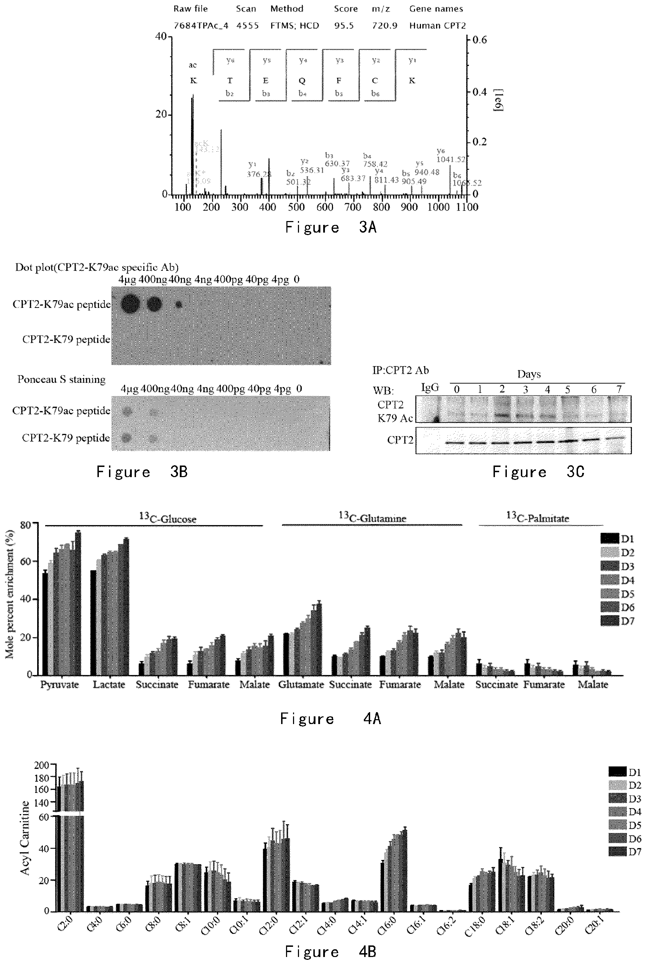

Figure 3A. Quantitative proteomic analysis of acetylation modification revealed that CPT2 was acetylated at lysine 79 in platelets.
Figure 3B. The acetylated polypeptides, but not nonacetylated polypeptides binds to CPT2K79AC antibody, verified by dot plot.
Figure 3C. By immunoprecipitation and western blot analysis, the changes of acetylation levels at position 79 in CPT2 increased sharply on the second day in stored platelets and decreased slowly during subsequent storage.
Figure 4A. By metabolic flux analysis of platelets, final metabolic fates of various concentrates were tracked using stable isotope labeling. It was found that during platelet storage, the metabolic efficiency of fatty acid was very low and declined with the increase of storage days; glucose mainly generates lactate through glycolysis pathway; glutamine is preferentially utilized, with a large amount entering the tricarboxylic acid cycle to provide energy for cells.
Figure 4B. Acylcarnitine content in plasma accumulates during platelet storage. During platelet storage, the samples were collected daily and plasma was collected for mass spectrometry analysis. After sample pretreatment by non-derivatization, stable isotope-labeled acylcarnitine was added as a standard, with ultra-high-performance liquid chromatography-triple quadrupole mass spectrometry system used for detecting more than 70 kinds of acylcarnitines.

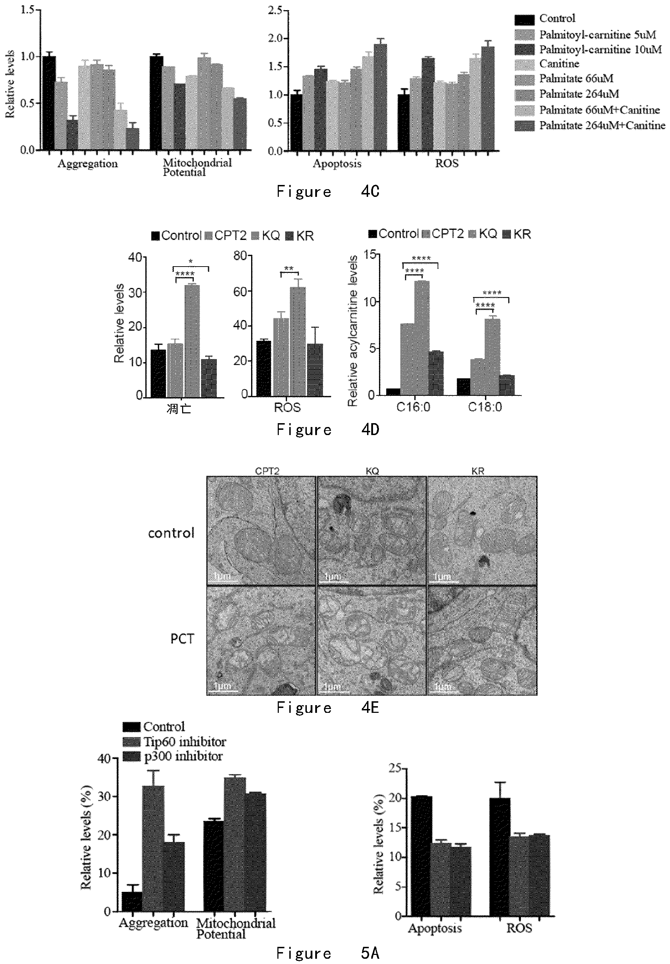

Figure 4C. The effects of palmitate, palmitic carnitine, and palmitoyl carnitine during platelet storage, as detected by flow cytometry. Reference indicators include platelet activity, fluorescence values of Annexin V and mitochondrial Mitosox.
Figure 4D. The acetylation at position 79 of CPT2 detected by liquid chromatography-tandem mass spectrometry can lead to the accumulation of acylcarnitine in cells. The accumulation of acylcarnitine affects cellular state. Flow cytometry analysis showed that with the increase of acylcarnitine concentration, the apoptosis ratio of cells and the level of reactive oxygen in mitochondria both increased.
Figure 4E. Transmission electron microscopy data showed that the mitochondria in CPT2 and KR cells were healthy and orderly arranged. However, the mitochondria in KQ cells exhibited disarrayed cristae and a decreased electron density. When cells were exposed to low concentrations of palmitoylcarnitine, the KR mitochondria was able to maintain its normal structure.
Figure 5A. Reducing the acetylation levels of proteins in platelets can effectively improve the quality of platelets. By adding two representative acetylase inhibitors to apheresis platelets, the effect of reduced acetylation of platelet proteins on platelet quality was observed. Regardless of whether it was the experimental group added with the tip60 inhibitor or the experimental group added with the p300 inhibitor, compared with the control group, the quality of the apheresis platelets stored for 7 days was significantly improved.

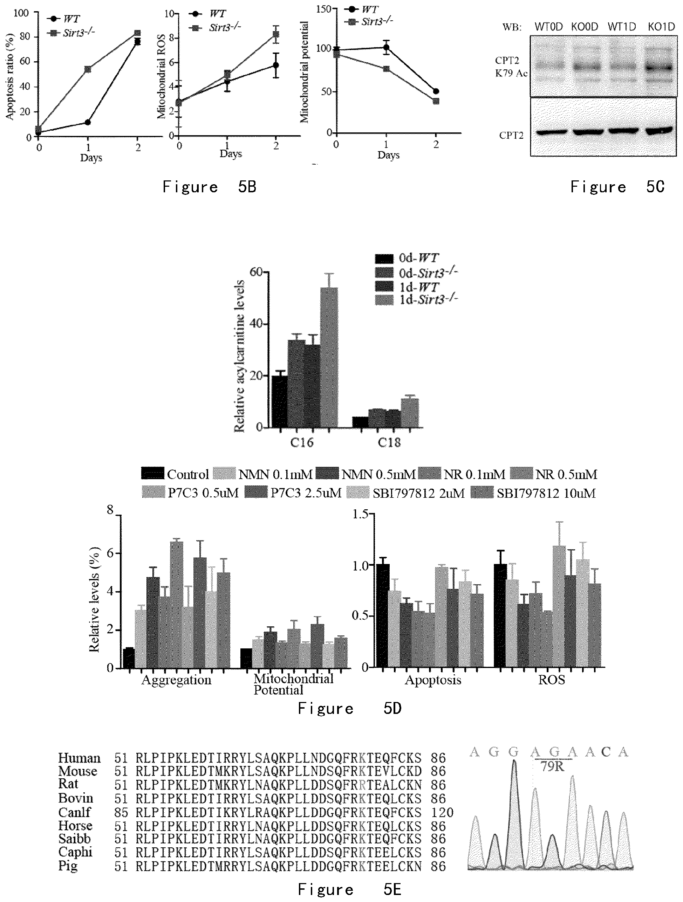

Figure 5B. Deacetylation of platelet proteins plays a critical role in platelet storage and aging deterioration. Platelets from SIRT3-knockout mice were more prone to apoptosis during storage than platelets from control mice and the mitochondrial membrane potential decreased faster, with more reactive oxygen accumulated during metabolism.
Figure 5C. SIRT3 knockout mice has a higher level of acetylation at position 79 of CPT2 compared to the wild-type mice. During platelet storage of mice, the level of acetylation of cpt2 79 increased, and the increased level of acetylation in sirt3 knockout mice was more significant.
Figure 5D. Derangement of fatty acid metabolism occurs in stored platelets of mice. As the storage time of platelets increases, the content of long-chain acylcarnitines inside the platelets continues to accumulate. Additionally, the degree of mitochondrial protein acetylation significantly affects the content of long-chain acylcarnitines within the platelets. The addition of four compounds improves the quality of platelets.
Figure 5E. Alignment of amino acid sequences of CPT2 molecules in various organisms shows that rat is a natural CPT2K79R model. Comparing the storage status of platelets in mice and rats, it can be found that rat platelets are more resistant to storage. Mouse platelets are more sensitive to acylcarnitine. Acylcarnitine can effectively damage mouse platelets, but rat platelets can withstand a certain concentration of acylcarnitine.

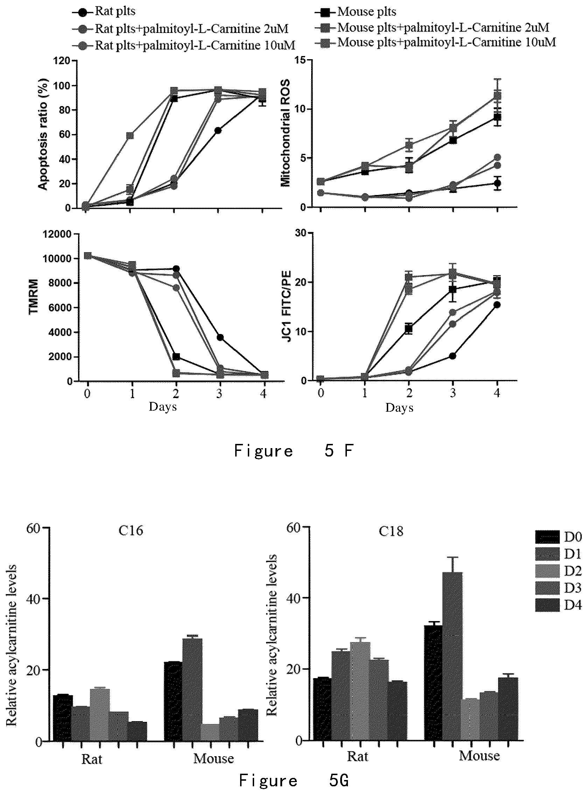

Figure 5F. The platelets in the rat CPT2K79R model can resist the accumulation of acylcarnitine in cells, ensuring the normal metabolism of fatty acid metabolism, and thus protecting cells from apoptosis.

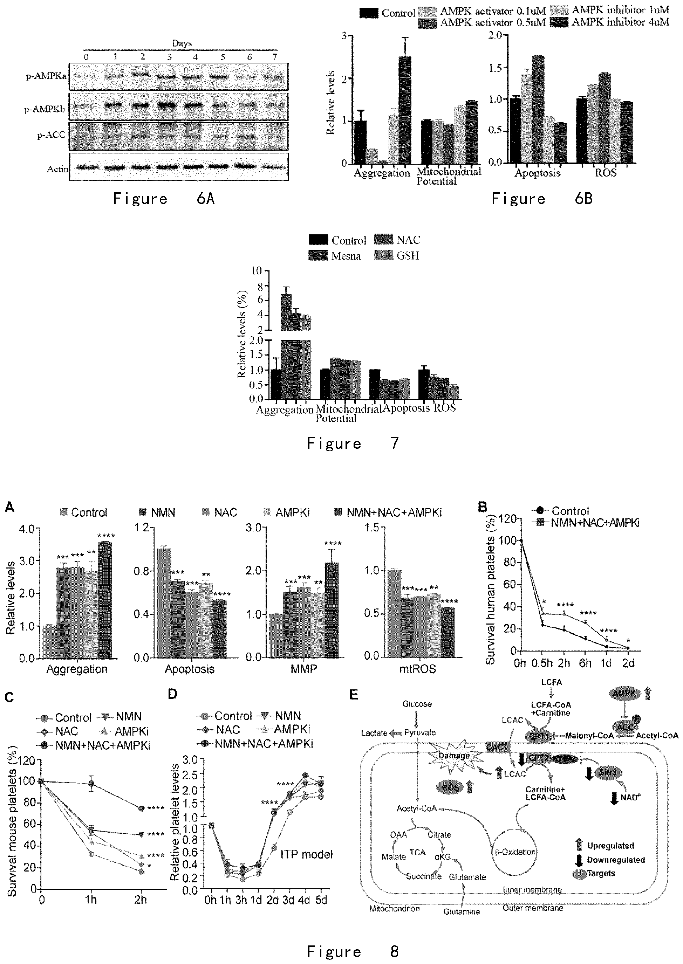

Figure 6A. Western blot analysis showed that during platelet storage, the AMPK-ACC signaling pathway was gradually activated, the abnormal energy metabolism of platelets triggered AMPK phosphorylation and phosphorylation-activated AMPK phosphorylated its downstream substrate ACC. Decreased phosphorylated ACC activities result in decreased fatty acid anabolic activities and increased catabolic activity.
Figure 6B. Effects of different concentrations of AMPK inhibitors and agonists on stored platelets. The activation and inhibition of AMPK pathway are closely related to the quality of platelet storage. By adding different concentrations of AMPK inhibitors and agonists to platelets, it is clear that the activation of the AMPK pathway is closely related to the apoptosis of stored platelets, mitochondrial potential difference, active oxygen accumulation and aggregation activity. Besides, this effect is concentration-dependent.
Figure 7. Small molecule inhibitors with antioxidant effects (acetylcysteine, mesna and glutathione) can reduce the accumulation of reactive oxygen in mitochondria, thereby preserving the responsiveness of platelets to stimulants and the mitochondrial potential difference. At the same time, the apoptosis ratio of stored platelets was also reduced.
Figure 8A. Combination of NAD+ precursor NMN, ROS scavenger NAC and AMPK inhibitor (AMPKi) (in freshly collected platelets, the addition of NMN is 0.5mM, the addition of NAC is 5mM, and the addition of AMPKi is 4uM) in improving the quality of stored platelets. This effect was much better than any of them, with reduced platelet apoptosis by half and increased platelet activity to about 4 times that of the control group.
Figure 8B. NMN+NAC+AMPKi (NMN 0.5mM, NAC 5mM, AMPKi 4uM) or PBS as a control was added. Human platelet concentrates were stored for 6 days in vitro. They were then labeled with biotin and infused into NCG mice. Platelet clearance was lower in the NMN+NAC+AMPKi group compared to the control group.
Figure 8C. Mouse platelet-rich plasma (PRP) was stored in vitro for 1 day, added with NMN, NAC, AMPK inhibitor or NMN+NAC+AMPK inhibitor (NMN 0.5mM, NAC 5mM, AMPKi 4uM) or PBS as a control. They were then labeled with biotin and infused into WT mice. Platelets stored with NMN, NAC, or AMPK inhibitor survived in vivo better than controls, and there was an additive effect of the three compound combinations.
Figure 8D. Evaluation of the effect of NMN, NAC or AMPK inhibitors on platelet survival in ITP patients. The ITP mouse model was established by tail vein injection of low-dose anti-GPIb antibody R300. The platelet survival rate and the recovery rate of platelet counts in the NMN, NAC or AMPK inhibitor group were higher than those in the control group, especially in the NMN+NAC+AMPKi group (1 µmol NMN, 10 µmol NAC, 8 nmol AMPKi).
Figure 8E. Schematic diagram of the intracellular mechanism involved in CPT2.

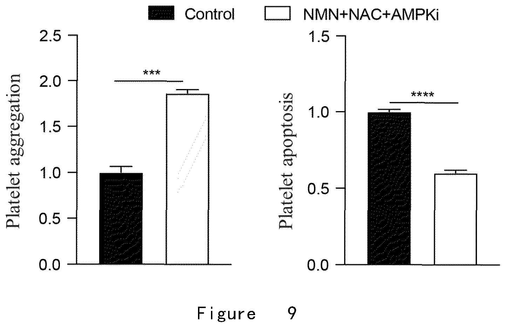

Figure 9. The aggregation activity of platelets added with NMN+NAC+AMPKi was significantly higher than that of the control group, and the apoptosis was significantly lower than that of the control group.

### DETAILED DESCRIPTION

After in-depth research, the inventors revealed for the first time that the molecular of carnitine palmitoyl transferase II (CPT2) of the platelet mitochondrial inner membrane is critical to the survival, preparation and storage of platelets, the reduction of CPT2 protein expression and/or activity seriously affects the quality of platelets, and the normal or high expression of the protein improves the quality of platelets. By means of directly and indirectly influencing or modifying CPT2 protein, the effect of CPT2 on platelets is verified at multiple angles, and a pathway and endogenous or exogenous molecules having a key effect of maintaining activity of platelets are disclosed. Therefore, the CPT2 itself or an up-regulator thereof can be used for maintaining the activity of platelets, and maintaining or improving storage quality of platelets, and can also be used for screening substances capable of maintaining the activity of platelets or improving storage quality thereof on the basis of the CPT2 or the signal pathway involving same and using same as a target. The present disclosure confirms that NAC, NMN, AMPK inhibitors alone and their combinations can effectively prolong the lifespan of platelets, enhance their activities and improve quality and life cycle of platelets both in vivo and in vitro by affecting various compounds of CPT2. These drugs have been clinically used, with guaranteed safety.

### CPT2

The amino acid sequence of human CPT2 can be shown as GenBank accession number 1376. The present disclosure also comprises CPT2 homologues from other species (such as primates, rodents; specifically monkeys, mice, etc.) and uses thereof.

The CPT2 of the present disclosure may be naturally occurring, for example, it may be isolated or purified from human or non-human mammals. The CPT2 can also be artificially prepared, for example, recombinant CPT2 can be produced according to conventional genetic engineering recombination for experimental or clinical applications. Recombinant CPT2 may be used in applications. The CPT2 comprises full-length CPT2 or a biologically active fragment thereof. According to the amino acid sequence of CPT2, corresponding nucleotide coding sequence of CPT2 can be easily obtained.

The amino acid sequence of CPT2 formed with substitution, deletion or addition of one or several amino acid residues is also included in the present disclosure. CPT2 or a biologically active fragment thereof comprises a sequence with substitution of several conservative amino acids, wherein the sequence with amino acid substitution does not affect its activity or retains part of its activity. Appropriate substitution of amino acids is a well-known method in the art. This method can be readily performed and ensures that the biological activity of the resulting molecule is not altered. These methods allow those skilled in the art to recognize that, in general, changes to single amino acids in non-essential regions of a polypeptide do not substantially alter its biological activity.

Any one of biologically active fragments of the CPT2 can all be applied to the present disclosure. Here, the biologically active fragment of CPT2 refers to a polypeptide that can still maintain all or part of the functions of the full-length CPT2. Usually, the biologically active fragments maintain at least 50% of the activity of the full-length CPT2. Under more preferable conditions, the active fragment can maintain 60%, 70%, 80%, 90%, 95%, 99%, or 100% of the activity of the full-length CPT2.

Modified or improved CPT2s can also be used in the present disclosure, eg, CPT2s that have been modified or improved to enhance their half-life, effectiveness, metabolism, and/or protein potency can be used. The modified or improved CPT2 may be a conjugate of CPT2, or it may comprise substituted or artificial amino acids. The modified or improved CPT2 may have less commonality with naturally occurring CPT2, but can also maintain or improve the activity of platelets, maintain or improve storage quality of platelets. That is, any variant that does not affect the biological activity of CPT2 can be used in the present disclosure.

### Uses of CPT2 and an up-regulator thereof

In the researches of the present inventors, it was unexpectedly found that during platelet metabolism, protein modifications and differential expressions derived from mitochondria, particularly acetylation of lysine 79 on carnitine palmitoyltransferase II (CPT2) within platelet mitochondria inner membrane, significantly influence platelet lifespan. Furthermore, the present disclosure confirms that CPT2 dysfunction, leading to inhibition of platelet fatty acid metabolism pathways, affects platelet quality through energy metabolism pathways (Figure 8E). In addition, the present disclosure further confirms that by directly or indirectly modulating the function of CPT2, such as directly using protein acetylation inhibitors, activating the deacetylase enzyme SIRT3 gene, or indirectly intervening in key signaling pathways of cellular energy metabolism pathways (such as the AMPK-ACC pathway, reactive oxygen accumulation), it is possible to protect platelets, particularly their quality.

Based on the new findings of the inventors, the present disclosure provides a use of CPT2 or an upregulation thereof in the preparation for: (1) maintaining or improving the activity of platelets; (2) maintaining or improving storage quality of platelets; or for use as a target for drug screening.

In the present disclosure, the up-regulator of CPT2 comprises but are not limited to: a substance capable of reducing acetylation of CPT2; a substance capable of increasing activity of CPT2; a substance capable of improving expression, stability or effective time of CPT2, and so on.

As used herein, the up-regulator of CPT2 comprises activators, agonists, and so on. Any substance that can increase the activity of CPT2, increase the stability of CPT2, up-regulate the expression of CPT2, promote the secretion of CPT2, increase the effective time of CPT2, or promote the transcription and translation of CPT2 can be used in the present disclosure as effective substances with upregulating functions.

As a preferable embodiment of the present disclosure, the up-regulator of CPT2 comprises (but not limited to): an expression vector or an expression construct capable of expressing (preferably over-expressing) CPT2 after being transferred into cells. Usually, the expression vector comprises a gene cassette, and the gene cassette comprises the gene encoding CPT2 and the regulatory sequences operatively linked thereto. The term "operably linked" or "operably linked to" refers to a condition that certain parts of a linear DNA sequence can regulate or control the activity of other parts of the same linear DNA sequence. For example, a promoter is operably linked to a coding sequence if it controls the transcription of the sequence.

In the present disclosure, the CPT2 polynucleotide sequence can be inserted into a recombinant expression vector, so that it can be transferred into cells and over-expressed to produce CPT2. Any plasmid and vector can be used as long as it can replicate and be stable in the hosts. An important characterastic of an expression vector is that it usually contains an origin of replication, a promoter, a marker gene, and translation control elements. For example, the expression vectors comprise: viral vectors, non-viral vectors; preferably, the expression vectors comprise (but are not limited to): adeno-associated virus, lentiviral vectors, adenoviral vectors and so on. Those skilled in the art are well known of methods for constructing expression vectors containing DNA sequences comprising CPT2 and suitable transcription/translation control signals. These include in vitro recombinant DNA techniques, DNA synthesis techniques, in vivo recombination techniques, etc.

According to the disclosure of the present disclosure, CPT2 acetylation occurs during platelet storage. Therefore, the up-regulator of the present disclosure is preferably an agent targeting acetylation site of CPT2 to reduce or abolish acetylation of the site. The acetylation site is lysine 79 of CPT2. Any agent capable of targeting and regulating this site of CPT2 or adjacent positions thereof, thereby reducing its acetylation level can be used in the present disclosure. Preferably, the agent comprises but are not limited to an agent for site-directed mutagenesis or site-directed modification targeting the lysine 79. The preparation of such agents for site-directed mutagenesis reagents or site-directed modification can be carried out relying on various methods in the art, such as but are not limited to gene editing, homologous recombination, etc. As another preferable embodiment of the present disclosure, protein acetylation inhibitors can be used to inhibit the acetylation of CPT2. The protein acetylation inhibitor may be a lysine acetyltransferase inhibitor, a mitochondrial protein acetylation inhibitor, or an activator or expression up-regulator thereof.

Lysine acetyltransferase (HAT) can acetylate a series of proteins to regulate the activity of proteins, and then regulate important cell functions such as cell apoptosis and cell metabolism. Lysine acetyltransferase catalyzes the transfer of the acetyl group of acetyl-CoA to the lysine of the substrate protein, thereby affecting the function of the substrate protein.

Both tip60 and p300 are members of the typical acetyltransferase family. tip60 belongs to the MYST acetyltransferase family. It plays an important regulatory role in the activation of cell cycle checkpoints, cell apoptosis and autophagy. At the same time, it can also play a role in various metabolic pathways such as glucose metabolism. p300 is an important class in the histone acetylase family, named p300 because of its molecular weight of 300kd. p300 plays a regulatory role in the process of cell proliferation and apoptosis. P300 and tip60 are expressed in mitochondria and can act as acetyltransferases in mitochondria, acetylating non-histone proteins and changing protein activities. According to the results of the examples of the present disclosure, the lysine acetyltransferase inhibitor (tip60 inhibitor or p300 inhibitor) can improve storage quality of platelets by inhibiting the acetylation of proteins in platelets. This demonstrates that reducing the acetylation levels of proteins in platelets can effectively improve the quality of platelets.

Mitochondrial protein acetylation inhibitors can also be used in the present disclosure. SIRT3 is a nicotinamide adenine dinucleotide (NAD+) dependent deacetylase, a member of the Sirtuin family, and a key protein deacetylase in mitochondria. It can act on mitochondrial proteins such as CPT2 to make it deacetylates. Results of the examples in the present disclosure show that platelets lacking SIRT3, due to the lack of deacetylation mechanism of mitochondrial proteins, lead to increased acetylation of mitochondrial proteins, thereby affecting the storage state of platelets. The acetylation level at position 79 of the CPT2 in SIRT3-knockout platelets is higher, with an influence on normal physiological functions of the CPT2, causing fatty acid metabolism disorders in platelets, and seriously inhibiting the quality of platelets. Moreover, the level of fatty acylcarnitine in SIRT3-knockout platelets is higher than that of the control group, indicating that with the increase of platelet storage time, the content of long-chain fatty acylcarnitine in platelets will continue to accumulate; and the acetylation level of mitochondrial proteins can significantly affect the content of long-chain fatty acylcarnitine in platelets. This indicates that the acetylation level of enzymes related to fatty acid metabolism in platelets may be altered, thereby affecting the fatty acid metabolism in platelet mitochondria.

According to the results in the examples of the present disclosure, the activator of the mitochondrial protein acetylation inhibitor SIRT3, that is, the agent that increases the concentration of NAD[+] precursor (nicotinamide (NAM), nicotinamide riboside (NR)) or an agent that increase NAMPT enzyme activity (P7C3, SBI-797812) can significantly improve the quality of platelets.

In the present disclosure, the correlation between key pathways of cell energy metabolism and CPT protein and platelet quality is also analyzed, indicating that inhibiting the activation of AMP-dependent protein kinase (AMPK) has a protective effect on platelet storage. AMPK has the function of regulating the energy metabolism of the body and maintaining the balance of energy supply and demand. When the intracellular energy level is reduced, the activation of AMPK can be triggered to regulate the metabolic pathways of oxidation and synthesis. Acetyl-CoA carboxylase (ACC) is one of the downstream targets of AMPK. Activated AMPK can phosphorylate ACC, thereby inhibiting its function, reducing the synthesis of malonyl-CoA and increasing the activity of CPT1 to promote fatty acid oxidation. Results of the examples of the present disclosure demonstrate that inhibiting the activation of the AMPK pathway in platelets can effectively preserve the quality of stored platelets, while inhibiting the activation of the AMPK pathway reduces the catalysis of fatty acid oxidation by CPT1, decreasing the entry of fatty carnitine into the mitochondria, thereby restoring the function of CPT2. AMPK pathway inhibitors can indirectly restore the protein function of CPT2. Therefore, as another preferable embodiment, AMPK pathway inhibitors can be used to improve the quality of platelets, for example comprising but not limited to: Dorsomorphin 2HCl.

In addition, antioxidants can reduce reactive oxygen accumulation and indirectly improve the function of CPT2. Therefore, antioxidants can also be used in the present disclosure; preferably comprising (but are not limited to): N-acetylcysteine, Mesna or glutathione.

It should be understood that limited but representative protein acetylation inhibitors, mitochondrial protein acetylation inhibitors, AMPK pathway inhibitors, and antioxidants are listed in the examples of the present disclosure. Other compounds or proteins that have the same or similar functions as these inhibitors can also be used in the present disclosure. Analogs, derivatives, isomers, precursors or salts of some compound inhibitors can also be used in the present disclosure.

In the examples of the present disclosure, specifically listed molecules that affect the quality of platelets are all endogenous drugs or existing clinical drugs, which are safe and accessible. Therefore, the target found in the present disclosure and its related influencing molecules can be used to improve the lifespan of stored platelets and platelets in the physiological microenvironment. At the same time, CPT2 as a target can be further used for genetic engineering of platelets.

The present disclosure also provides a pharmaceutical composition, wherein it comprises effective amounts (eg, 0.000001-20wt%; preferably 0.00001-10wt%) of the CPT2, or an up-regulator thereof, or an analogue thereof, and a pharmaceutically acceptable carrier.

The composition of the present disclosure can be directly used to improve the quality of platelets (including maintaining or improving the activity of platelets, maintaining or improving storage quality of platelets). In addition, it can also be used in combination with other active agents or adjuvants at the same time.

Generally, these materials can be formulated in a non-toxic, inert medium with pharmaceutically acceptable aqueous carrier, wherein the pH is usually about 5-8, preferably the pH is about 6-8.

As used herein, the term "comprise" means that various components can be used together in the mixture or composition of the present disclosure. Therefore, the term "mainly consist of......" and "consist of......" is included in the term "comprise". As used herein, the term "effective amount" refers to an amount that is functional or active for humans and/or animals and is acceptable for administration to humans and/or animals.

As used herein, "pharmaceutically acceptable" ingredients refer to substances suitable for use in humans and/or mammals without undue adverse side effects (such as toxicity), i.e. with reasonable benefit/risk ratio. The term "pharmaceutically acceptable carrier" refers to a carrier for the administration of a therapeutic agent, comprising various excipients and diluents.

The composition of the present disclosure comprises a safe and effective amount of CPT2, or an up-regulator thereof (such as an expression vector that overexpresses the CPT2), or an analog thereof, and a pharmaceutically acceptable carrier. Such excipients include (but are not limited to): saline, buffer, glucose, water, glycerol, ethanol, and combinations thereof. Usually, the pharmaceutical formulation should match the route of administration. The pharmaceutical composition of the present disclosure can be prepared in the form of an injection, for example, using a saline solution or a water solution containing glucose and other excipients through conventional methods. The pharmaceutical composition should be manufactured under sterile conditions. The amount of active ingredient administered is a therapeutically effective amount. The pharmaceutical formulations of the present disclosure can also be prepared into sustained-release formulations.

After knowing uses of the CPT2, various methods well known in the art can be used to administer the CPT2 or an coding gene thereof, or a pharmaceutical composition thereof. The administration method of the CPT2 or an up-regulator thereof can be determined according to actual needs, mainly depends on the type and characteristics of the up-regulator, which can be evaluated by those skilled in the art. When the CPT2 is a compound, it can be administered systemically or locally for platelets in the body; for platelets treated in vitro, it can be added to the platelets to be treated or plasma comprising platelets.

When the CPT2 is a gene or protein, for example, CPT2 or an up-regulator thereof can be directly administered to the subject through methods such as injection. Alternatively, expression unit carrying the CPT2 gene (such as an expression vector or virus, etc.) can be delivered to the target t, allowing active expression of active CPT2. As an embodiment of the present disclosure, the CPT2 can be directly administered to mammals (such as humans), or the gene encoding CPT2 can be cloned into an appropriate vector (such as conventional prokaryotic or eukaryotic expression vectors, or virus vectors such as herpes virus vectors or adenovirus vectors) by conventional methods, then the vectors can be introduced into cells that can express the CPT2, so that the cells express CPT2.

### CPT2 as a drug screening target

After knowing the function and mechanism of CPT2, substances that promote the expression or activity of CPT2 can be screened based on this characteristic, and the substances can be used as candidate substances for improving the quality of platelets. The candidate substances can be but are not limited to: overexpressed molecules for example constructs, active promoters, chemical small molecules, interacting molecules targeting CPT2, fragments or variants thereof, encoding genes thereof or upstream or downstream molecules or molecules in signaling pathway.

In a preferable embodiment of the present disclosure, when performing screening, a control group may be established to facilitate the observation of changes in the expression or activity of CPT2 and the signaling pathway proteins involved, or upstream and downstream proteins thereof. The control group can be a system expressing CPT2 or upstream and downstream signaling pathways thereof without the addition of the candidate substance.

The expression system may be, for example, a cell (or cell culture) system, and the cells may be cells that express CPT2 endogenously; or cells that express CPT2 recombinantly. The system for expressing CPT2 can also be (but are not limited to) a subcellular system, a solution system, a tissue system, an organ system, an animal system (such as an animal model), and so on.

As a preferred embodiment of the present disclosure, the method also comprises: further cell experiments and/or animal experiments on the obtained substances (potential substances), so as to further select and determine substances really useful for improving activity or storage quality of platelets.

The method for detecting the expression, activity, amount or secretion of CPT2 or upstream and downstream proteins thereof is not particularly limited in the present disclosure. Conventional protein quantitative or semi-quantitative detecting methods can be used, such as (but not limited to): SDS-PAGE method, Western-Blot method, ELISA and so on.

On the other hand, the present disclosure also provided compounds, compositions or drugs, or some potential substances obtained by the screening method. Some initially screened substances can constitute a screening library, so that people can finally screen out substances that are really useful for improving activity or storage quality of platelets and useful in clinical.

### CPT2 as a target for assessing platelet quality

Based on the new findings of the present disclosure, CPT2 can be used as a marker for assessing platelet activity or storage quality: assessing/detecting platelet activity or platelet storage quality. Especially for the detection of its acetylation site, preferably the site is lysine 79.

Various methods known in the art can be used to detect the presence and expression conditions, expression levels or activities of the CPT2 gene, and these methods are all included in the present disclosure. For example, existing methods such as Southern blotting, Western blotting, DNA sequence analysis, PCR, and other methods can be used, and these methods can be used in combination.

The present disclosure also provides an agent for detecting the presence or absence and expression of the CPT2 gene in the analyte. Preferably, when performing detection at the gene level, primers for specific amplification of CPT2 can be used; or probes for specific recognition of CPT2 can be used to determine the presence or absence of the CPT2 gene. When performing detection at the protein level, an antibody or ligand that specifically binds the protein encoded by CPT2 can be used to determine the expression of CPT2 protein. As a preferable embodiment of the present disclosure, the agent is a primer, which can specifically amplify the CPT2 gene or gene fragment. The design of specific probes for the CPT2 gene is a method well known to those skilled in the art, for example, preparing a probe that can specifically bind to a specific site on the CPT2 gene, but not other genes except the CPT2 gene, and the probe has a detectable signal. In addition, the method of detecting the expression of CPT2 protein in an analyte using an antibody that specifically binds to CPT2 protein is also well known to those skilled in the art.

In this field, it is a mature technology to measure the acetylation level of the acetylation site of protein. In general, two commonly used methods can be used to determine the acetylation site: one is directly purifying target proteins in cells for mass spectrometry identification; another is using purified target proteins and acetyltransferase in vitro for in vitro acetylation reaction, with the target protein after the reaction identified by mass spectrometry. No matter which method determines the acetylation site, it is generally necessary to construct mutants for final determination. Acetylation mainly occurs on lysine (K). Generally speaking, mutation of lysine to arginine (R) is equivalent to mimic deacetylation, and mutation of lysine to glutamine (Q) is equivalent to mimic acetylation.

Once the acetylation site is determined, the simulated acetylation/deacetylation mutant can generally be used to study the effect of acetylation on the function of the target protein. In general, researches on acetylation modification of proteins generally comprise the following steps: determining whether the target protein has acetylation; screening acetylases/deacetylases; identifying acetylation sites; analyzing the impact of acetylation on the target proteins; investigating biological functions of protein acetylation.

The present disclosure also provides a kit for detecting the presence or absence and expression of the CPT2 gene in an analyte, comprising: an agent for specifically detecting the acetylation level of the acetylation site of the CPT2 protein; primers for specific amplification of CPT2 gene; probes for specific recognition the CPT2 gene; or antibodies or ligands for specifically binding the CPT2 protein.

In addition, the kit may also comprise various reagents required for DNA extraction, PCR, hybridization, color development, etc., including but not limited to: extraction solution, amplification solution, hybridization solution, enzyme, control solution, chromogenic liquid, lotion, etc. Additionally, the kit also comprises instructions for use and/or nucleic acid sequence analysis software.

### Genetically engineered platelets or precursors thereof

The present disclosure also provides a genetically engineered platelet or a precursor thereof, wherein there is an exogenous carnitine palmitoyltransferase II or an up-regulator thereof converted in the platelet or the precursor. Preferably, the up-regulator of carnitine palmitoyltransferase II is an agent targeting acetylation site of carnitine palmitoyltransferase II to reduce or abolish acetylation of the site; more preferably, the agent targeting the acetylation site of carnitine palmitoyltransferase II, lysine 79, making it mutated for reducing or abolishing the acetylation.

The carnitine palmitoyltransferase II or an up-regulator thereof according to the present disclosure can be converted in platelets from various sources. Platelets isolated from human body, fibroblasts or platelets differentiated from megakaryocyte stem cells are included. The stem cells may comprise hematopoietic stem cells, embryonic stem cells, and induced stem cells, which can be induced to differentiate in vitro to produce mature functional platelets. Platelet production is a result of the synergistic interaction of various cytokines in the bone marrow stromal microenvironment. Generally speaking, a relatively complete platelet culture system in vitro can be obtained by simulating the microenvironment of platelet production in vivo.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### General methods

### 1. Generation of CPT2K79Q point mutation mice

### 1.1 CPT2K79Q/R mice

The amino acid sequence of CPT2 is UniProtKB-P23786.

Generation of CPT2K79Q mice: Using C57BL/6J strain as background, a point mutation was carried out on the CPT2 gene at EXON3 (K79Q: AAG→CAG). A target site ending with NGG near this site was designed, with a 90 bp donor cut and constructed in vitro. The exogenous donor was inserted into the cleavage site by homologous recombination. The above experimental protocol for model mouse construction was provided by Yaobang Biological Company.

Sequence analysis was used for genotype identification of offspring. Sequencing with forward primers, base mutation at the target sites was checked by the electropherogram. The sequences of guide RNA and gene are as follows:
CPT2-sgRNA2: tgtgttttcttgaaaattacagG(SEQ ID NO: 1)
CPT2-sgRNA3: GAAGACAGAAGTGTTGTGTAAGG(SEQ ID NO: 2)
CPT2(K79Q)-Oligo2:
   gtggtttgacctgtgttttcttgaaaattacagACAGACAGAGGTGTTGTGTAAGGATTTTGAGAACGGCAT TG*G*G(SEQ ID NO: 3)
CPT2(K79Q)-Oligo2:
   gtggtttgacctgtgttttcttgaaaattacagAAGGACAGAGGTGTTGTGTAAGGATTTTGAGAACGGCAT TG*G*G(SEQ ID NO: 4)
1.2 CPT2K79R mice was generated by similar methods, with a difference of K79R: AAG-AGG point mutation. The sequences for base mutation identification on guide RNA and gene are as follows:
CPT2-sgRNA2: tgtgttttcttgaaaattacagG(SEQ ID NO: 5)
CPT2-sgRNA3: GAAGACAGAAGTGTTGTGTAAGG(SEQ ID NO: 6)
CPT2(K79Q)-Oligo2:
   gtggtttgacctgtgttttcttgaaaattacagAAGGACAGAGGTGTTGTGTAAGGATTTTGAGAACGGCAT TG*G*G(SEQ ID NO: 7)

### 2. SIRT3 knockout mice

The background of the SIRT3 knockout mouse strain is C57BL/6N. The SIRT3 knockout mice were obtained by high-throughput electroporation. The mice are housed under SPF (Specific Pathogen Free) conditions, with ad libitum access to water and food. The humidity of the housing environment is maintained between 40-70% and the temperature is controlled around 23°C. The mice are subjected to a simulated 12-hour light-dark cycle, managed and housed by the Department of Animal Sciences at Shanghai Jiao Tong University School of Medicine.

### 3. Mouse genotype identification

The mice were numbered, with the tails cut, and the genomic DNA of the mice was extracted by absolute ethanol. After PCR amplification, the Gel Image System 2500 gel imaging system was used for gel imaging by ultraviolet irradiation.

### PCR primers

| | | |
|---|---|---|
| Primers for SIRT3KO mice genotype identification | SIRT3 WT primer: scttctgcggctctatacacag | SEQ ID NO: 8 |
| | SIRT3 Common primer: tgcaacaaggcttatcttcc | SEQ ID NO: 9 |
| | SIRT3 Mutant primer: tactgaatatcagtgggaacg | SEQ ID NO: 10 |
| Primers for identification on CPT2 point mutation of mice | Forward primer: ctttcctcggtgcctctgt | SEQ ID NO: 11 |
| | Reverse primer: cttcccaatgccgttctca | SEQ ID NO: 12 |

### PCR reaction system

| Reagent | Volume |
|---|---|
| 10×PCR Buffer | 5µl |
| dNTPs(2mM) | 5µl |
| MgSO4(25mM) | 3µl |
| KOD-Plus polymerase (1U/µl) | 1µl |
| Mouse tail DNA (100-200µg/ml) | 1µl |
| Primer 1 (10µM) | 1.5µl |
| Primer 2 (10µM) | 1.5µl |
| Primer 3 (10µM) | 1.5µl |
| Deionized water | Added to 50µl |
| Total volume | 50µl |

### PCR reaction conditions

| | Temperature | Time |
|---|---|---|
| Pre-denaturation | 94 °C | 2min |
| Denaturation | 98°C | 10s |
| Annealing | SIRT3-KO mice identification: | 30s |
| | Identification on CPT2 point mutation of mice: 54°C | |
| Extension | 68°C | SIRT3-KO mice identification: 30s |
| | | Identification on CPT2 point mutation of mice: 17s |

| Repeated denaturation-annealing-extension for 35 cycles. | | |
|---|---|---|
| Post extension | 68°C | 3min |
| Cooling | 4°C | HOLD |

### PCR products were sequenced and genotype-identified.

PCR products of the CPT2 point mutant mouse were sequenced together with the forward primer, and the returned base sequence was identified and confirmed with 4PEAKS software.

SIRT3-KO mice genotype identification: A single 500bp band indicates that the mouse is wild type; a single 100bp band indicates that the mouse is a knockout type. If there are bands of 100bp and 500bp at the same time, it indicates that the mouse is heterozygous.

### 4. Storage at room temperature and sample collection of human apheresis platelets

An appropriate amount of apheresis platelets was added to a 50ml centrifuge tube (the volume should not exceed 15ml/tube). The mouth of the 50ml centrifuge tube was covered and fixed with a clinical platelet storage bag as a gas-permeable membrane, placed on a horizontal shaker. The speed of the shaker was determined according to the volume inside the tube, ensuring the platelets were fully shaken and fully exposed to oxygen for respiration without touching the gas-permeable membrane.

This method can simulate the storage of clinical apheresis platelets in a laboratory environment and can be used for sampling and the sampling is completed. The collected apheresis platelet samples can be used for platelet counting, flow cytometry index detection, protein collection, platelet viability detection, ATP content detection and other purposes.

### 5. Storage of mouse platelets

Blood was taken from the abdominal aorta of mice. Apyrase (in a final concentration of 1 U/ml) and PGE1 (in a final concentration of 0.1 µg/ml) that can inhibit platelet aggregation was then added with to the whole blood, mixed quickly and centrifuged (1050rpm, room temperature, 10 minutes). After two centrifuges, the upper plasma of the experimental group was sucked out, discarded and the platelet precipitate at the bottom was retained. The plasma of wild-type mice was evenly distributed to the platelet pellets of mice in the experimental group and wild-type group. The platelets were uniformly dispersed and suspended, and then preserved according to the method of human platelets.

### 6. Detection of platelet aggregation

By conventional methods, using a platelet aggregation instrument, 300 µl of 3×10⁸ platelets/ml and the required inhibitors were added to the aggregation tube and incubated at 37°C for 3 minutes after gentle stirring. A baseline was established and the changes of an aggregation curve were observed within 5 minutes.

### 7. Platelet apoptosis

Platelets were sampled according to conventional methods, then the concentration of platelets was diluted to 10^8/ml with Tyrodes's Buffer. 25 µl of platelets in the experimental group and control group were added to new EP tubes. Each tube was added with 5 µl of 10×Binding Buffer, 2 µl of FITC labeled Annexin V and 18µl of Tyrodes's Buffer to a total of 50µl in the reaction system. After incubating at room temperature in the dark for 15 minutes, 200 µl 1×Binding Buffer was added for detection on the machine, with a tube of unlabeled platelets as a negative control. The platelets labeled with Annexin V were detected within one hour after labeling and the FITC-positive cell population was the platelets in the process of apoptosis.

### 8. Flow cytometric detection of apoptosis in stable cell lines

Adherent cells were digested with trypsin and collected, centrifuged at 2000rmp at 4°C for 5 minutes and washed several times with PBS (1ml PBS was added to the cell pellet and placed for 3 minutes, centrifuged at 2000rmp at 4°C for 5 minutes) to completely remove the residual EDTA. After centrifugation, the supernatant was removed and the cells were resuspended, with 5 µl of fluorescently labeled Annexin V added and mixed. After incubating for 15 minutes at room temperature in the dark, 5 µl of propidium iodide was added for continuous incubation for 5 minutes at room temperature in the dark. 200µl×Binding Buffer was added for detection on the machine. The detection was completed within 1 hour.

### 9. Flow cytometry for detecting mitochondrial potential difference

### 9.1 Changes in platelet mitochondrial potential difference were detected by JC1 fluorescent dyes:

A JC1 working solution was prepared according to conventional methods. Positive control platelets were treated with CCCP to reduce mitochondrial membrane potential, that is, the positive control. Sampled platelets were diluted to 10⁸/ml. 25 µl of platelets was taken and added with 25 µl of JC1 working solution. After mixed and incubated at 37°C in the dark for 30 minutes, a 200 µl of 1×JC1 staining buffer was added for detecting the fluorescence intensity on the machine. The relative ratio of red and green fluorescence was used to represent the ratio of mitochondrial depolarization.

### 9.2 Changes in platelet mitochondrial potential difference were detected by TMRM fluorescent dyes

The platelet sampling and dilution were as described above. 50 µl Tyrodes's Buffer and 0.1 µl TMRM stock solution were added to a 50 µl dilution solution, incubated at 37°C in the dark for 30 minutes, with 500 µl Tyrodes's Buffer added for detection on the machine. The fluorescence intensity of TMRM will red shift according to the change of mitochondrial potential difference. The fluorescence intensity was used to represent the level of mitochondrial potential difference.

### 9.3 Changes of adherent cells in platelet mitochondrial potential difference were detected by TMRM fluorescent dyes

The cells were stably plated one day in advance and different concentrations of acylcarnitine or other inhibitors were added to different cells according to the experimental requirements. The next day, TMRM was diluted with cell culture medium at a ratio of 1:1000 to prepare a working solution. The prepared cells were replaced with medium and placed in an incubator for 30 minutes. The cells were washed twice with ice-cold PBS, digested with trypsin and collected, centrifuged at 600g for 5 minutes to obtain a cell pellet. The cells were washed twice with PBS, resuspended in 200µl PBS and tested for fluorescence on the machine.

### 10. Detection of Mitosox fluorescent dyes

Using the conventional method, 25 µl platelets were diluted in equal proportions, added with 0.1 µl Mitosox reagent (5 mM) and incubated in the dark at 37°C for 30 minutes, with 200 µl buffer added to detect the fluorescence intensity; stained the adherent cells: after centrifugation at 600g for 5 minutes to remove the supernatant, the cells were resuspended in 200 µl Mitosox working solution, incubated at 37°C in the dark for 30 minutes, centrifuged at 600 g for 5 minutes, washed twice with PBS and added with 200 µl PBS for resuspending and detecting the cells.

### 11. CPT2, CPT2K79Q and CPT2K79R plasmid and stable cell lines

A plasmid of pLVX-IRES-ZsGreen1 inserted with CPT2K79R, a plasmid of pLVX-IRES-ZsGreen1 inserted with CPT2K79Q, and a plasmid of pLVX-IRES-ZsGreen1 inserted with CPT2 were synthesized by Shanghai Sunny Biotechnology Co., Ltd. Lentiviral transfection, 293T cell infection, and stably transfected cell lines were all carried out by conventional methods.

### 12. Measurement of ATP levels in platelets

ATP level was determined using a commercial kit. Platelets (3×10⁸) were collected and mixed with EDTA and Apyrase, then centrifuged to obtain a platelet pellet. Then 200µl of lysis buffer for detecting ATP level was added to this pellet, with the platelets dispersed, and the mixture was kept on ice during the procedure. After centrifugation at 12,000g for 5 minutes at 4°C, the supernatant was collected for subsequent analysis. Standardization was performed by diluting ATP standard solutions to concentrations of 10, 3, 1, 0.1, and 0.01µM/l. The reagent for detecting ATP level and the diluted reagent for detecting ATP level were mixed in a ratio of 1:9. ATP concentration was determined as follows: 100µl of working solution for detecting ATP level was added to each well and placed at room temperature for 3-5 minutes. Subsequently, 20µl of different concentrations of standard solutions and testing samples were added to the wells of an opaque 96-well plate, mixed rapidly, and the fluorescence value was measured in a luminometer. A standard curve was used to calculate the ATP concentration in the testing samples. Additionally, the remaining testing samples were subjected to BCA protein quantification to eliminate measurement errors caused by differences in protein concentrations.

### 13. Mitochondrial Mitotracker staining

Mitotracker Deep Red FM-special packaging was purchased from Thermofisher. Experimental cells were prepared one day in advance by placing circular glass coverslips at the bottom of a 24-well plate and seeding the cells. The plate was then incubated overnight at 37°C in a cell culture incubator to ensure that the cell density in each well was between 50% and 70% the next day. The cell culture medium in the 24-well plate was removed and the cells were washed twice with PBS. Next, Mitotracker working solution (1mM Mitotracker Deep Red diluted to a working concentration of 100nM) was added to the wells of the 24-well plate. The plate was then returned to the cell culture incubator at 37°C and incubated for an additional 30 minutes. After staining, the cells were washed with PBS and gently agitated on a horizontal shaker for 5 minutes. The PBS buffer was then replaced with fresh PBS, and this washing step was repeated twice. Each well was then fixed with 200µl of 4% formaldehyde at room temperature for 10 minutes. After fixation, the cells were washed with PBS again. DAPI stain (ready-to-use) was added to each well of the 24-well plate, and the plate was incubated at room temperature for 10 minutes in dark. A drop of anti-fade reagent was added to a microscope slide. Then the slide was carefully removed from the 24-well plate, blotted dry. Images of the slides were captured from five random fields using a fluorescence microscope, with different fluorescence channels overlaid.

### 14. Seahorse metabolic analyzer used for monitoring real-time mitochondrial aerobic metabolism and glycolysis in platelets and stable transfected cell lines

Adherent cells were seeded at a ratio of 30,000 cells per well. If drug effects on cellular respiration needed to be assessed, inhibitors were added simultaneously during cell seeding. Celltak was used as a cell adhesive to coat the cell culture plate, allowing suspended cells to adhere to the bottom of the Seahorse XF96 cell plate. On the day of the experiment, an appropriate volume of platelet suspension was added to each well. For assessing glycolytic capacity, 10⁷ platelets were seeded into the experimental wells. For measuring cellular oxygen consumption, 2×10⁷ platelets were seeded into the experimental wells and allowed to adhere to the Celltak-coated surface.

Two Seahorse detecting buffers were mixed as follows:

| OCR detecting buffer | | ECAR detecting buffer | |
|---|---|---|---|
| Glutamine 100× | 1ml | Glutamine 100× | 1ml |
| Glucose | 0.2g | XF Assay Medium | 99ml |
| Pyruvate100× | 1ml | | |
| XF Assay Medium | 98ml | | |

While in a water bath at 37°C, the pH was adjusted to 7.4 with 1mM sodium hydroxide and the mixture was placed in a water bath at 37°C for later use.

Drug preparation and dosing methods in mitochondrial stress kit and glycolysis stress kit
1) A dosing scheme for detecting platelet metabolism

| | Drug powder | Volume of detecting buffer (ml) | Original Concentration | Working Concentration (Final Concentration) | Concentratio ns in wells with drugs | Scheme for diluting stock solutions |
|---|---|---|---|---|---|---|
| ECAR | Glucose | 3 | 100mM | 10mM | 100mM | No dilution needed |
| | Oligom ycin | 0.75 | 100µM | 1µM | 10µM | 10-fold dilution from stock solution |
| | 2-DG | 3 | 500mM | 50mM | 500µM | No dilution needed |
| OCR | Oligom ycin | 0.63 | 100µM | 2µM | 20µM | 5-fold dilution from stock solution |
| | FCCP | 0.72 | 100µM | 0.25µM | 2.5µM | 40-fold dilution from stock solution |
| | Rotenon e/ Antimy | 0.54 | 50µM | 1µM | 10µM | 5-fold dilution from stock solution |
| | cina | | | | | |

2) A dosing scheme for detecting stable transfected cell metabolism

| | Drug powder | Volume of detecting buffer(ml) | Original Concentration | Working Concentration (Final Concentration) | Concentrations in wells with drugs | Scheme for diluting stock solutions |
|---|---|---|---|---|---|---|
| ECAR | Glucose | 3 | 100mM | 10mM | 100mM | No dilution needed |
| | Oligomycin | 0.75 | 100µM | 1µM | 10µM | 10-fold dilution from stock solution |
| | 2-DG | 3 | 500mM | 50mM | 500µM | No dilution needed |
| OCR | Oligomycin | 0.63 | 100µM | 1µM | 10µM | 10-fold dilution from stock solution |
| | FCCP | 0.72 | 100µM | 0.5µM | 5µM | 20-fold dilution from stock solution |
| | Rotenone/ Antimycina | 0.54 | 50µM | 0.5µM | 5µM | 10-fold dilution from stock solution |

The hydrated testing plate was taken from a carbon dioxide-free incubator, and drugs with diluted concentrations corresponding to each drug-dosing well were added to three drug-dosing wells of A/B/C, of wherein 20 µl was added to well A, 22 µl was added to well B and 25 µl was added to well C.

### 15. Preparation of bovine serum albumin and stable isotope-labeled palmitate conjugates

2.267g of fatty acid-free bovine serum albumin was used and added to 100ml of 150mM NaCl solution under stirring. The mixture was placed in a water bath at 37°C to maintain the temperature. Another 17mM BSA Control stock solution was prepared and stored at -20°C. 30.6 mg of stable isotope-labeled palmitate was added to 44 ml of 150 mM NaCl solution, stirred in a water bath at 70° C. Another 50ml of BSA solution was used and added to 40ml of palmitate solution in 8 times, stirred in a water bath at 37°C for more than 1 hour. The mixture was then adjusted to a volume of 100ml with 150mM NaCl solution, with the pH adjusted to 7.4 for preparing a palmitate conjugate in a concentration of 1mM with bovine serum albumin and stable isotope-labeled palmitate, stored at -20°C.

Apheresis platelets were freshly collected, with stable isotope-labeled palmitate (in a final concentration of 66 µM) added to the platelets. In the experimental group added with palmitate, L-carnitine was also added with a concentration ten times that of the palmitate (in a final concentration of 660µM). During storage, samples were taken every day and samples of platelets stored for 0-7 days were collected. 3×10⁸ platelets were obtained every day with the addition of EDTA and Apyase to inhibit platelet aggregation and activation. After centrifugation at 800g for 5 minutes at room temperature, the supernatant was discarded and a buffer was used for washing the platelets. Then a second centrifugation was performed. The platelet pellets were quickly frozen by quickly putting into liquid nitrogen, so as to quickly terminate the metabolism in the platelets. Stable isotope-labeled platelet samples were analyzed by Fluxomics and the morphology of mitochondria and changes in the internal structure of mitochondria were observed under a transmission electron microscope.

### 16. Construction of mouse ITP model

WT mice were intravenously injected with NMN, NAC, AMPK inhibitor, or NMN+NAC+AMPK inhibitor. After 30 minutes, basal platelet counts were first determined from complete blood counts using a HEMAVET automated hematology analyzer. Mouse platelets were cleared by intravenous injection of 2 µg anti-CD42b monoclonal antibody R300 (purchased from Emfret). Orbital blood was collected at different time points to monitor platelet clearance and recovery.

### 17. Refrigerated storage and sample collection of human apheresis platelets

An appropriate amount of apheresis platelets was added to a 50ml centrifuge tube (the volume should not exceed 15ml/tube). The mouth of the 50ml centrifuge tube was covered and fixed with a clinical platelet storage bag as a gas-permeable membrane, stored in a 4-degree refrigerator for 3 days. Platelet aggregation activity was detected and platelet apoptosis level was analyzed by flow cytometry.

### 18. Data analysis

All of the above experiments were statistically analyzed and plotted using Graphpad Prism 6 software. The data results were expressed in MEAN±SD. The P value was calculated by the Students' Test. The smaller P value indicates more significant result. P value less than 0.05 indicates the data with statistical significane.

### Example 1. The quality of platelets stored for different days declines as time increases

Freshly-collected apheresis platelets were stored in a standard clinically-used disposable platelet storage bag at room temperature (with a material of butyryl tri-n-hexyl citrate plasticized soft polyvinyl chloride calendered film) (purchased from Sichuan Nangeer Company). A horizontal shaker was arranged in an aseptic operating table in the laboratory cell room to simulate clinical storage conditions of apheresis platelets. After storage under standard conditions for 0 to 7 days, apheresis platelets stored for different days were collected every day, tracing the function and metabolism of platelets.

Experiments have found that platelets will gradually lose their ability to respond to activators during storage. Besides, when stored for 3-4 days, there is a rapid decline in platelet responsiveness to stimuli. At the same time, the mitochondrial potential of platelets also decreased with the increase of storage time. As an indicator of apoptosis , the exposure level of phosphatidylserine increases continuously during storage. Platelet aggregation to the activator also decreased significantly on the 4th day. Reactive oxygen species (ROS) levels in platelets exploded on day 3 of storage (Figure 1A).

By Western Blot analysis of Caspase3, it was found that platelets activated Caspase3 during storage, triggering the caspase-mediated apoptosis pathway, resulting in cell apoptosis. The activation of Caspase3 in platelets also showed an increasing trend with the increase of storage time. Protein abundance of Caspase3 cleavage represents the Caspase3 activation (Figure 1B).

It can be seen that platelets lose their responsiveness to activators, undergo increased apoptosis, accumulate reactive oxygen species, and experience mitochondrial depolarization during storage, with a rapid transition occurring after storage for 3-4 days. Further investigation into the metabolic and apoptotic processes of platelets is needed to elucidate the underlying factors behind these phenomena and to intervene effectively.

### Example 2. Differential expression of proteins derived from mitochondria leads to decreased platelet quality

### 1. Acetylation quantitative proteomics study of platelet samples found that the acetylation level of the protein changed.

Platelet samples stored for 0 to 6 days were collected. Quantitative proteomics on acetylation of platelets was studied by combining a series of techniques such as TMT labeling, high performance liquid chromatography fractionation and mass spectrometry-based quantitative proteomics.

First, the cells were lysed and the proteins were extracted. Proteins were digested into peptides with trypsin, labeled with TMT and then fractionated by high performance liquid chromatography, followed by liquid chromatography-mass spectrometry tandem analysis. The obtained secondary mass spectrometry data were searched by Maxquant (V1.5.2.8). Finally, bioinformatics analysis was performed on the data. A total of 3,204 proteins with quantitative information were identified in this experiment, with 1.3 times used as the threshold and T-Test P-Value<0.05 as the standard. Among the quantified proteins, there are 14 up-regulated proteins and 6 down-regulated proteins in the 1d/0d comparison group. In the 2d/0d comparison group, there are 7 up-regulated proteins and 6 down-regulated proteins. In the 3d/0d comparison group, there are 28 up-regulated proteins and 21 down-regulated proteins. In the 4d/0d comparison group, there are 41 up-regulated proteins and 225 down-regulated proteins. In the 5d/0d comparison group, there are 28 up-regulated proteins and 11 down-regulated proteins. In the 6d/0d comparison group, there are 38 up-regulated proteins and 16 down-regulated proteins. Further analysis of acetylation modification on platelets with altered expression found that when platelets were stored for 3-4 days under conventional storage conditions, the number of proteins with acetylation in platelets increased rapidly (Figure 2A).

### 2. Determine the protein localization of acetylation modification

Further bioinformatical analysis was carried out on proteins with altered acetylation levels, including detailed analysis of KEGG pathways and subcellular structural localization.

Using Wolfpsort software, the subcellular structure classification and statistics of acetylated differentially expressed proteins on the 4th day of storage were analyzed, wherein up to a quarter of protein species are originated from mitochondria (Figure 2B). It indicates that on the fourth day of platelet storage, large amounts of proteins of mitochondria showed acetylation modification.

After enriching differential proteins in different comparison groups of KEGG pathway, the inventors further performed cluster analysis on these proteins, aiming to find a functional correlation of differential proteins in the comparison groups. Based on the enrichment test P value obtained from the enrichment analysis, the hierarchical clustering method was used to cluster related functions in different groups together, with a heat map drawn. The horizontal part of the heat map represents the enrichment test results of different comparison groups, and the vertical part represents the description of related functions. Color blocks corresponding to the differentially expressed proteins and functional descriptions in different comparison groups indicate the degree of enrichment. A redder color indicates a stronger degree of enrichment. A bluer color indicates a weaker degree of enrichment. Analysis of the heat map showed that platelet activation pathway and fatty acid metabolism-related pathways were highly enriched, indicating that related proteins of these two pathways were regulated by acetylation modification during platelet storage (Figure 2C).

### Example 3. Reduction of CPT2 molecular activity may be related to the reduction of platelet quality

In Examples 1-2, the proteomic analysis of human platelets was carried out. It was found that in both analyses, the acetylation level of a large number of enzymes related to fatty acid metabolism changed with storage time. After further analysis, the inventors found that carnitine palmitoyltransferase II (CPT2) was acetylated with time during storage, and the acetylation occurred at lysine 79 (CPT2K79), indicating that the modification of lysine 79 of CPT2 protein can obviously change with storage time, and the acetylation of lysine 79 can alter CPT2 functions (Figure 3A).

By synthesizing peptide fragments comprising acetylated lysine 79 of CPT2, the inventor prepared an antibody specific to the CPT2 with acetylation at position K79. As verified by dot plot, the acetylated polypeptides, but not nonacetylated polypeptides binds to the antibody (Figure 3B). The antibody was used to detect the level of acetylation at position 79 of CPT2 in human platelet samples stored from 0 days to 7 days. Through the detection of immunoprecipitation and Western Blot, it was found that acetylation of CPT2 did occur during storage. Moreover, acetylation level at position 79 of the CPT2 increased with the storage time, and reached the peak of acetylation on the second day of platelet storage, and then the acetylation at position 79 of CPT2 decreased slowly (Figure 3C).

### Example 4. Fatty acid metabolism disorder caused by CPT2 dysfunction directly affects the quality of platelets

Considering that the CPT2 is located in the inner mitochondrial membrane, it is an essential catalytic enzyme in the fatty acid oxidation process, catalyzing the splitting of fatty acylcarnitine into fatty acyl-CoA and carnitine, and transporting fatty acids into mitochondria to prepare for subsequent fatty acid oxidation. It is suggested that the reduction of CPT2 protein activity may affect the quality of platelets through energy metabolism.

### 1. Blockage of fatty acid metabolic pathway in platelets

Glutamine and fatty acids are three important energy sources for the body. The inventors used liquid chromatography-mass spectrometry to conduct an experiment of C13 tracking metabolic pathways. A C13-labeled palmitate and carnitine, a C13-labeled glucose and C13-labeled glutamine were added to professional platelet storage bags capable of gas exchange.

In the experimental group added with stable isotope-labeled palmitate and carnitine, the metabolism of fatty acids was significantly inhibited, and the labeling rate of intermediate metabolites in the tricarboxylic acid cycle decreased with the increase of storage days, indicating that with the increase of storage days, fewer fatty acids are oxidized into the tricarboxylic acid cycle. The fatty acid oxidation pathway is impaired during platelet storage. On the 7th day of storage, the labeling rate of malate and fumarate was less than 10%. This indicated that the rate of fatty acid oxidative breakdown also decreased during platelet storage (Figure 4A).

### 2. Among the fatty acid metabolism, due to CPT2 dysfunction, long-chain acylcarnitine (C16:0, C18:0) accumulates over time.

Fatty acids are essential energy sources for the body. In the cytoplasm, fatty acids are converted into fatty acyl-CoA by acyl-CoA synthetase. The long chain acyl-CoAs cannot pass through the mitochondrial membrane freely. They must be converted to acyl-carnitines by carnitine palmitoyltransferase 1 (CPT1) located on themitochondrial outer membrane to form acylcarnitine in association with carnitine, enabling entry into the mitochondrial matrix for further oxidation. After entering the mitochondria, acylcarnitines are catalyzed back to acyl-CoAs and carnitines by CPT2 acyl-CoA Acyl-CoAs enter the mitochondrial matrix and are sequentially catalyzed to form acetyl-CoA through β-oxidation for entering the TCA cycle and eventually producing energy.

After observing the obstruction of fatty acid metabolism within platelets, the levels of acylcarnitine intermediates were tracked using liquid chromatography-mass spectrometry. It was found that long-chain acylcarnitines (C16:0, C18:0) accumulated in platelet plasma, with an increase in plasma acylcarnitine levels as storage time increased. The level of acylcarnitines in plasma reflects the state of fatty acid metabolism. Free fatty acids must undergo metabolism to acylcarnitines before they can be transported across the mitochondrial membrane by transporters and enter the mitochondria for further fatty acid oxidation to provide cellular energy. The accumulation of acylcarnitines in platelets prior to storage indicates highly active fatty acid metabolism, with a large amount of fatty acid metabolism preparing for complete oxidation to provide energy. However, the reduced activity of CPT2 protein prevents long-chain fatty acids from entering the mitochondria, hindering energy metabolism. The abnormal accumulation of long-chain fatty acid acylcarnitines within cells suggests a correlation between platelet fatty acid metabolism disorders and impaired catalytic function of CPT2 (Figure 4B).

### 3. The increase in acylcarnitine concentration poses a risk to platelet storage.

The phenomenon of acylcarnitine accumulation within platelets corresponds to the increase in ROS and apoptosis observed in platelets stored for 3-4 days. Therefore, it can be speculated that this harmful fatty acid intermediate metabolite, acylcarnitine, triggers metabolic disorders through some mechanisms, leading to cell apoptosis.

To elucidate the detrimental effects of acylcarnitine on platelets, palmitate, carnitine, palmitate combined with carnitine and palmitoylcarnitine were added separately to freshly isolated platelets. On the second day of platelet storage, platelet activity was measured, and a series of mitochondria-related parameters were assessed, including mitochondrial membrane potential and mitochondrial reactive oxygen species levels. Flow cytometry analysis revealed that abnormal elevation of acylcarnitine concentration is detrimental to platelet storage. Cells treated with palmitoylcarnitine exhibited higher apoptosis rates, lower mitochondrial membrane potential, and higher levels of mitochondrial reactive oxygen species compared to the control group. Additionally, platelet status was significantly worse in the experimental group treated with both fatty acids and carnitine simultaneously than the groups treated with carnitine alone or palmitate alone. This further illustrates the detrimental effects of palmitoylcarnitine, an intermediate metabolite produced from the reaction between palmitate and carnitine, on platelet storage. Acylcarnitine is thus a highly harmful fatty acid intermediate metabolite (Figure 4C).

### 4. The effect of CPT2K79 acetylation on acylcarnitine

To further verify the correlation between CPT2K79 acetylation and the accumulation of acylcarnitine, three plasmids were constructed: wild-type CPT2, CPT2K79R mutation, and CPT2K79Q mutation. These three plasmids were packaged into lentiviruses using a three-plasmid system for infecting 293T cells. The exogenous genes were integrated into the host genome, and four stable transfected cell lines were selected through flow cytometry sorting. CPT2K79R is a CPT2 molecule with the mutation of lysine at position 79 to arginine, abolishing acetylation at position 79. CPT2K79Q is a CPT2 molecule with the mutation of lysine at position 79 to glutamine, simulating acetylation at position 79.

Subsequently, various parameters of the four stable transfected cell lines were studied. Liquid chromatography-mass spectrometry was used to detect the content of acylcarnitine in the four cell lines. It was found that compared to the empty vector control group, the CPT2K79Q mutant cell line accumulated more acylcarnitine, while the CPT2K79R cell line had significantly lower acylcarnitine content than the CPT2K79Q cell line.

Fluorescence staining was performed on the four stable transfected cell lines, and flow cytometry results revealed that the state of the CPT2K79Q cell line was significantly worse than that of the CPT2K79R cell line. Specifically, the CPT2K79Q cell line showed higher levels of reactive oxygen species accumulation and apoptosis rates compared to the other three stable transfected cell lines, while the CPT2K79R cell line exhibited lower levels of reactive oxygen species accumulation and apoptosis ratio than the other three stable transfected cell lines (Figure 4D).

The experiment demonstrated that mutation at position 79 of the CPT2 significantly affected the accumulation of acylcarnitine, cell apoptosis ratio and mitochondrial reactive oxygen species accumulation. Acetylation at position 79 of the CPT2 inhibits its function. Acetylated CPT2 at position 79 cannot catalyze the cleavage of acylcarnitine into acyl-CoA and carnitine, leading to the accumulation of acylcarnitine within cells. At the same time, the accumulation of acylcarnitine affects cellular state. Acylcarnitine, as a toxic metabolic intermediate, is often used clinically as an indicator of fatty acid metabolism disorders. As the concentration of acylcarnitine accumulates, the apoptosis ratio of cells and the level of reactive oxygen species within mitochondria increase.

Above experiments confirm that CPT2 dysfunction in the CPT2K79Q cell line leads to the accumulation of acylcarnitine. Conversely, the CPT2K79R cell line exhibits the opposite effect. However, CPT2 dysfunction inevitably leads to the inability of acylcarnitine to undergo subsequent metabolism, resulting in its accumulation within mitochondria.

After treating CPT2 and CPT2 mutant stable transfected cell lines with acylcarnitine for 4 hours, the cells were fixed. Then electron microscopy was used to observe the status and structure of mitochondria in different cell lines treated with or without palmitoylcarnitine. Compared to the three groups of cells without palmitoylcarnitine, structural abnormalities were observed in the mitochondria of the CPT2K79Q cell line. Specifically, there were anomalies in the morphology and arrangement of mitochondrial cristae, wherein the cristae became disarrayed, fragmented and blurred, with bright areas appearing in the center of the mitochondria, indicating severe damage to the mitochondrial inner membrane structure. The mitochondria of the CPT2K79R cell line and the CPT2 cell line exhibited neatly arranged and clear cristae structures, with fewer bright areas, indicating healthy mitochondrial states in these cell lines.

After incubating the three cell lines with low concentrations of palmitoylcarnitine for 4 hours, it was evident that the mitochondria in the group treated with palmitoylcarnitine were significantly worse than those in the group without palmitoylcarnitine. In the cells treated with palmitoylcarnitine, the mitochondrial inner membrane further vacuolized, and fusion of vacuoles resulted in the formation of multi-vesicular structures. Additionally, numerous electron-dense phagosomes were observed in cells treated with palmitoylcarnitine, possibly indicating lysosomes after phagocytosis of damaged mitochondria, further illustrating severe mitochondrial damage. It is noteworthy that the low-dose palmitoylcarnitine treatment had little effect on the mitochondrial status of the CPT2K79R cell line. Even with the addition of palmitoylcarnitine, the mitochondria in the CPT2K79R cell line did not exhibit multi-vesicular cristae structures. The effect of palmitoylcarnitine on the mitochondrial status is particularly pronounced in the CPT2K79Q cell line with CPT2 dysfunction, and to a lesser extent in the CPT2 cell line (Figure 4E).

### Based on the above experiments, the following conclusions can be drawn:

1. Palmitoylcarnitine exhibits toxicity to mitochondria, which can affect the morphology of cellular mitochondria and consequently influence cellular metabolic capacity.
2. In CPT2K79Q cells, structural acetylation of the CPT2 leads to a decrease in the catalytic ability, resulting in the accumulation of more acylcarnitine within mitochondria and subsequent changes in mitochondrial morphology and decreased metabolic capacity. With the additional supplementation of acylcarnitine, CPT2 cannot metabolize acylcarnitine, exacerbating mitochondrial damage.
3. In CPT2K79R cells, where the CPT2 cannot undergo acetylation, the catalytic ability of CPT2 remains stable. These cells exhibit less accumulation of acylcarnitine within mitochondria, leading to well-maintained mitochondrial morphology. Even with additional supplementation of acylcarnitine, CPT2 retains sufficient activity to metabolize acylcarnitine, ensuring that the mitochondrial structure is not damaged even under high acylcarnitine concentrations.

### Example 5. Effects of directly changing CPT2 functions on platelet quality

As deduced from the preceding experiments, it is evident that in the process of declining platelet quality, there is an abnormal expression of CPT2. The effect of CPT2 on platelet metabolic pathways demonstrates that abnormalities in CPT2 lead to disturbances in fatty acid metabolism, ultimately affecting platelet quality. To further explore methods for enhancing CPT2 functions and ensuring platelet quality, the following experiments were conducted.

### 1. Effects of protein acetylation inhibitors on platelet quality

Lysine acetyltransferase (HAT) can acetylate a series of proteins to regulate the activity of proteins, and then regulate important cell functions such as cell apoptosis and cell metabolism. Lysine acetyltransferase catalyzes the transfer of the acetyl group of acetyl-CoA to the lysine of the substrate protein, thereby affecting the function of the substrate protein.

Both tip60 and p300 are members of the typical acetyltransferase family. tip60 belongs to the MYST acetyltransferase family. It plays an important regulatory role in the activation of cell cycle checkpoints, cell apoptosis and autophagy. At the same time, it can also play a role in various metabolic pathways such as glucose metabolism. p300 is an important class in the histone acetylase family, named p300 because of its molecular weight of 300kd. p300 plays a regulatory role in the process of cell proliferation and apoptosis. P300 and tip60 are expressed in mitochondria and can act as acetyltransferases in mitochondria, acetylating non-histone proteins and changing protein activities.

In fresh isolated apheresis platelets, inhibitors of tip60 and p300 were added, and the platelets were stored under standard storage conditions for 7 days. After 7 days, various parameters of the stored apheresis platelets were measured, including platelet responsiveness to agonists, degree of platelet apoptosis, mitochondrial membrane potential, and levels of mitochondrial reactive oxygen species.

The experiment revealed that after adding acetyltransferase inhibitors, whether inhibitor of p300 or Tip60, the quality of apheresis platelets was effectively improved by inhibiting the acetylation of proteins within platelets. Compared to the control group, the experimental groups treated with tip60 inhibitor (10 uM) and p300 inhibitor (10 uM) significantly enhanced platelet responsiveness to agonists, increased mitochondrial membrane potential, reduced platelet apoptosis and decreased levels of mitochondrial ROS (Figure 5A). This indicates that inhibiting protein acetylation within platelets can enhance storage quality of apheresis platelets, suggesting a close correlation between the acetylation level of proteins within platelets and storage quality of platelets. Reducing the acetylation levels of proteins in platelets can effectively improve the quality of platelets.

### 2. Effects of mitochondrial protein acetylation inhibitors on platelets

Silent mating type information regulation 2 homolog-3 (SIRT3) is a nicotinamide adenine dinucleotide (NAD+) dependent deacetylase, a member of the Sirtuin family, and a key protein deacetylase in mitochondria. It can act on mitochondrial proteins such as CPT2 to make it deacetylates. This experiment investigated the effects of SIRT3 on platelets.

### (1) SIRT3 knockout mice exhibit reduced platelet quality

Firstly, an ex vivo storage experiment was conducted using platelets from SIRT3 knockout mice. Platelet pellets from wild-type mice and SIRT3 knockout mice were prepared in a sterile environment, and then the platelets were resuspended with plasma from wild-type mice to avoid interference of plasma factors on platelet storage. Platelet-rich plasma from mice was stored under standard clinical apheresis platelet storage conditions, with samples taken daily to assess platelet status based on parameters including platelet apoptosis rate, mitochondrial membrane potential, and accumulation of mitochondrial reactive oxygen species.

The experiment revealed that compared to wild-type mice in the control group, platelets from SIRT3 knockout mice exhibited faster apoptosis, increased accumulation of ROS within mitochondria, and a more rapid decline in mitochondrial membrane potential during storage (Figure 5B). This confirms that platelets lacking SIRT3, due to the lack of deacetylation mechanism of mitochondrial proteins, lead to increased acetylation of mitochondrial proteins, thereby affecting the storage state of platelets. This further validates the potentially crucial role of protein acetylation/deacetylation in platelet storage and aging deterioration.

### (2) SIRT3 knockout mice exhibit higher acetylation levels at position 79 of CPT2

Compared with the platelets of wild-type mice, the level of acetylation at position 79 of the CPT2 in platelets of SIRT3 knockout mice was higher. After 24 hours of storage, the level of acetylation at position 79 of the CPT2 in the platelets of wild-type mice and SIRT3-knockout mice increased, and the level of acetylation at position 79 of CPT2 in SIRT3-knockout mice increased significantly. Due to the absence of SIRT3 within platelets, platelets from knockout mice could not effectively undergo deacetylation modifications during storage, resulting in a significant increase in acetylation levels at position 79 of the CPT2 (Figure 5C). This affects the normal physiological function of the CPT2, leading to impaired fatty acid metabolism in platelets and a significant reduction in platelet quality.

### (3) Increased carnitine in platelets from SIRT3 knockout mice

Platelet samples were collected at day 0 and day 1 of storage in mice, and the carnitine in platelets from wild-type and SIRT3 knockout mice was measured. Long-chain carnitines (palmitoyl carnitine and stearoyl carnitine) were primarily detected.

The results showed that the carnitine in platelets from wild-type mice was significantly lower than that in platelets from SIRT3 knockout mice. Furthermore, after storage, the levels of long-chain carnitines increased compared to those in fresh platelets, with the level in platelets from SIRT3 knockout mice higher than that in the control group. This indicates that the content of long-chain carnitines continues to accumulate with increasing platelet storage time. Additionally, the level of mitochondrial protein acetylation significantly affects the content of long-chain carnitines in platelets (Figure 5D, upper). This suggests that changes in the acetylation levels of enzymes related to fatty acid metabolism in platelets can affect fatty acid metabolism within platelet mitochondria, consistent with the results of impaired CPT2 enzyme activity and further validating the correlation between CPT2 acetylation, fatty acid metabolism disorders, and impaired platelet quality.

### (4) Effects of activating SIRT3 on platelet quality

SIRT3 is an NAD(+) dependent deacetylase. The enzymatic activity of SIRT3 is regulated by the cellular levels of NAD+ and NADH. When cellular NAD+ level increases, SIRT3 activity is activated. On the contrary, when the NADH level increases, SIRT3 activity is inhibited.

Nicotinamide (NAM), as a precursor of nicotinamide adenine dinucleotide (NAD+), increases cellular NAD+ levels, thereby enhancing SIRT3 activity. Therefore, NAM serves as a SIRT3 activator. Adding NAM can indirectly up-regulate the activity of SIRT3 deacetylation. Nicotinamide phosphoribosyltransferase (NAMPT) is a rate-limiting enzyme catalyzing nicotinamide adenine dinucleotide (NAD+) synthesis.

Nicotinamide riboside (NR) is also a precursor of NAD[+]. Nicotinamide riboside increases NAD[+] levels by catalyzing with nicotinamide riboside kinase. Elevated NAD[+] levels can activate both SIRT1 and SIRT3, ultimately reducing mitochondrial protein acetylation and enhancing oxidative metabolism.

P7C3 enhances NAMPT activity by binding to NAMPT, thereby increasing cellular NAD+ levels through the NAD salvage synthesis pathway.

SBI-797812 is a compound structurally similar to nicotinamide phosphoribosyltransferase (NAMPT) inhibitors. It can block the binding of NAMPT inhibitors to NAMPT. SBI-797812 shifts the NAMPT reaction equilibrium towards NMN formation, increases NAMPT affinity for ATP, stabilizes phosphorylated NAMPT at His247, promotes the consumption of pyrophosphate by-products and inhibits NAD+ deactivation through feedback inhibition.

To validate the ability of these substances to directly or indirectly activate SIRT by increasing intracellular NAD levels, fresh apheresis platelets were taken and divided into the groups with NMN (0.1mM, 0.5mM), NR (0.1mM, 0.5mM), P7C3 (0.5uM, 2.5uM), and SBI797812 (2uM, 10uM) added to platelets to study the effects of these four compounds with the ability of increasing NAD levels on platelet quality.

On the 7th day of platelet storage, platelets were sampled and their apoptosis ratio, mitochondrial membrane potential, and levels of mitochondrial ROS were analyzed by flow cytometry. Platelet aggregation was also used to assess the platelets' responsiveness to agonists in different experimental groups.

The results showed that compared to the control group, the experimental groups supplemented with the four compounds (both high and low concentrations) exhibited improved platelet quality, and this improvement was concentration-dependent. The higher the concentration of the added small molecule compound, the greater the improvement of platelet quality. Specifically, improvements were observed in improved platelet responsiveness to agonists, elevated mitochondrial membrane potential and reduced apoptosis ratio and ROS levels (Figure 5D, lower).

This indicates that increasing NAD+ levels, whether by raising precursor concentrations or enhancing NAMPT enzyme activity, can improve platelet quality to varying levels. This also underscores the crucial importance of SIRT3 activity for quality of platelet preservation. Reducing the acetylation levels of mitochondrial proteins in platelets can effectively improve the quality of platelets.

### 3. CPT2K79 acetylation

Previous results indicated that CPT2K79 acetylation plays a crucial role in the expression and function abnormalities of CPT2 protein, thereby affecting platelet quality. Therefore, several amino acid sequences of CPT2 in animals and plants were compared, revealing that CPT2 is a conserved protein in mammals. In most mammals (such as humans and mice), the 79th position of the CPT2 protein is lysine (K), whereas in rats, it is arginine (R), indicating that rats naturally carry the CPT2 K79R mutation (Figure 5E). Consequently, it is validated that whether the level of rat platelets are affected by carnitine.

Whole blood was obtained from wild-type rats and wild-type mice via abdominal aortic puncture, and platelet-rich plasmas (PRP) were prepared separately for rats and mice. Platelet storage experiments were conducted, with two additional experimental groups each receiving the addition of palmitoyl carnitine at different concentrations (2µM and 10µM). Platelet apoptosis ratio, mitochondrial ROS level and changes in mitochondrial membrane potential were measured during storage days 0-4.

The experiment revealed that during storage, rat platelets exhibited lower apoptosis ratios and mitochondrial ROS levels compared to mouse platelets, while the mitochondrial membrane potential was higher than that of mice, indicating that the status of rat platelets was significantly better than that of mice (Figure 5F). Comparison of the results from days 1 and 2 showed that the addition of carnitine during platelet storage did not cause significant changes in the status of rat platelets. However, the addition of carnitine effectively reduced the status of mouse platelets, and this effect showed a concentration-dependent trend.

During the storage of wild-type rat and mouse platelets, samples were taken daily for liquid chromatography-mass spectrometry to detect the carnitine level. Analysis of the trends in palmitoyl carnitine and stearoyl carnitine changes revealed that the carnitine level in fresh rat platelets was significantly lower than that in fresh mouse platelets, indicating that the mutation of the 79th position of the CPT2 in rats can enhance CPT2 catalytic activity. During storage, the carnitine level in rat platelets remained stable, with slight accumulation by the third day of storage. However, the carnitine level in mouse platelets fluctuated dramatically with increasing storage time, sharply increasing by the second day of storage, followed by a rapid decrease in most platelets due to apoptosis (Figure 5G). It can be observed that the platelets in the rat CPT2K79R model can resist the accumulation of acylcarnitine in cells, ensuring the normal metabolism of fatty acid metabolism, and thus protecting cells from apoptosis.

This experimental result once again confirms the close relationship between acetylation at the 79th position of the CPT2 protein and the accumulation of carnitine, which is crucial for platelet quality. Inhibiting acetylation at the 79th position of the CPT2 protein can delay platelet apoptosis. Therefore, controlling the amino acid at position 79 of the CPT2 protein plays a key role in platelet quality.

### Example 6. Correlation between key pathways of cell energy metabolism and CPT protein and platelet quality

AMP-dependent protein kinase (AMPK) has the function of regulating the energy metabolism of the body and maintaining the balance of energy supply and demand. When the intracellular energy level is reduced, the activation of AMPK can be triggered to regulate the metabolic pathways of oxidation and synthesis.

Acetyl-CoA carboxylase (ACC) is the rate-limiting enzyme in fatty acid synthesis. Acetyl-CoA generated from glucose metabolism can be converted to malonyl-CoA by ACC. Malonyl-CoA, the first product of fatty acid synthesis, can inhibit activity of carnitine palmitoyltransferase 1 (CPT1) through negative feedback, thereby suppressing mitochondrial fatty acid oxidation. ACC is one of the downstream targets of AMPK. Activated AMPK can phosphorylate ACC, thereby inhibiting its function, reducing the synthesis of malonyl-CoA and increasing the activity of CPT1 to promote fatty acid oxidation (Figure 6A).

The research in this embodiment found that during platelet storage, ATP is rapidly consumed, leading to an increase in the ADP/ATP ratio and subsequent activation of AMPK. Western blot experiments confirmed the phosphorylation and activation of AMPK in platelets. One of the downstream substrates of AMPK, acetyl-CoA carboxylase, was also phosphorylated. Reduced ACC activity due to phosphorylation led to a decrease in the concentration of its metabolic product, malonyl-CoA. This decrease relieved the inhibitory effect of malonyl-CoA on CPT1, allowing CPT1 to catalyze large amounts of acylcarnitines into the mitochondria and accumulated, leading to CPT2 dysfunction.

To verify the effect of AMPK activation on platelets, AMPK inhibitor Dorsomorphin 2HCl (1uM, 4uM) and AMPK agonist AICAR (0.1uM, 0.5uM) were added to platelets. Flow cytometry was used to detect differences in platelet indicators between the experimental and control groups on the 4th day of storage.

The results showed that in the experimental groups treated with AMPK inhibitors, there were varying degrees of improvement in platelet responsiveness to stimuli, apoptosis ratio, mitochondrial reactive oxygen species levels and mitochondrial membrane potential, indicating a protective effect of inhibiting AMPK activation on platelet storage. However, in the experimental groups treated with AMPK agonists, it was found that the activation of AMPK pathway was not only detrimental to platelet responsiveness to stimuli, , but also increased apoptosis ratio. At the same time, it adversely affected mitochondrial functions, as evidenced by a significant decrease in mitochondrial membrane potential and elevation of mitochondrial reactive oxygen species levels (Figure 6B).

Simultaneously, the experimental results exhibited a clear concentration-dependent effect of AMPK agonists or inhibitors. The results demonstrate that inhibiting the activation of the AMPK pathway in platelets can effectively protect the quality of stored platelets, while inhibiting the activation of the AMPK pathway reduces the catalysis of fatty acid oxidation by CPT1, decreasing the entry of fatty carnitine into the mitochondria, thereby alleviating the burden on CPT2. Consequently, AMPK pathway inhibitors can indirectly influence the protein function of CPT2, reducing the accumulation of its product, acylcarnitines.

### Example 7. Antioxidants can reduce reactive oxygen accumulation and indirectly improve the function of CPT2.

In previous experiments, the level of reactive oxygen species (ROS) was considered one of the indicators of platelet quality. Considering the close association between cell apoptosis and ROS accumulation, it was hypothesized that inhibiting the generation and accumulation of ROS in platelets could enhance the quality of stored platelets and reduce the apoptosis ratio. To verify this hypothesis, antioxidants were added to freshly isolated apheresis platelets, and on the 7th day of storage, ROS level, apoptosis ratio and mitochondrial membrane potential of platelets were measured.

N-acetylcysteine (NAC) is a commonly used antioxidant. As a compound containing a thiol group, NAC has the function of scavenging and inhibiting the synthesis of oxygen free radicals. After entering cells, NAC can generate cysteine, wherein the cysteine promotes the synthesis of glutathione (GSH), the most important weapon against oxidative stress of the body. Additionally, NAC can directly inhibit cell apoptosis by inhibiting the release of cytochrome C, thereby suppressing the activation of Caspase3.

Mesna (sodium 2-mercaptoethane sulfonate) is a widely used chemoprotective agent clinically. This drug exhibits significant peroxidase activity and can also protect the antioxidant activity of superoxide dismutase (SOD) and glutathione (GSH).

Glutathione (GSH) has antioxidant and detoxifying effects. The structure of glutathione contains a reactive thiol group (-SH) that is easily oxidized and dehydrogenated. This group makes GSH an important scavenger of free radicals in the body, protecting cysteine residues in proteins and enzymes.

According to the experimental results, compared to the control group, three small molecule antioxidant inhibitors, N-acetylcysteine (at 5mM), Mesna (at 50uM), and glutathione (at 200uM), could all reduce the accumulation of intracellular reactive oxygen species to varying degrees. Furthermore, in the experiments with added antioxidant small molecule inhibitors, platelet responsiveness to stimuli and mitochondrial membrane potential were protected. Meanwhile, the apoptosis ratio of platelets in the experimental group was lower than that in the control group (Figure 7). This experiment further demonstrates that reducing the level of oxygen free radicals has a significant improvement effect on platelet quality.

### Example 8. Combination of CPT2 up-regulator, CPT1 inhibitor, and antioxidant significantly improves platelet survival in vitro and in vivo

Previous experiments have demonstrated that NAD+ depletion, CPT2 K79 acetylation, AMPK activation, acylcarnitine, and mtROS accumulation are the main features of platelet storage damage. The NAD+ precursor NMN, ROS scavenger NAC, and AMPK inhibitor (AMPKi) significantly improved the quality of stored platelets individually. Therefore, the combination of NMN, NAC, and AMPK inhibitor deserves further investigation into its effect on platelet storage damage.

First, their effects on the platelet storage were studied. The results shown in Figure 8A indicate that the combination of NMN, NAC, and AMPK inhibitor greatly improves the quality of stored platelets compared to any of them alone, resulting in a halving of platelet apoptosis and approximately a four-fold increase in platelet activity compared to the control group.

In platelet transfusions, it is undesirable for stored platelets to be rapidly cleared from circulation. To evaluate the effects of the above compounds on the quality of platelets stored in vivo, two mouse transfusion models were used. In the first model, stored human platelets were transfused into immunodeficient NCG (NOD-Prkdcem26Cd52I12rgem 26Cd22/NjuCrl) mice. NMN+NAC+AMPKi (NMN 0.5mM, NAC 5mM, AMPKi 4uM) or PBS as a control was added. Human platelet concentrates were stored for 6 days in vitro. They were then labeled with biotin and infused into NCG mice. Platelet counts significantly decreased within the first 0.5 hours. However, platelet clearance was lower in the NMN+NAC+AMPKi group compared to the control group (Figure 8B). In another group of models, mouse PRP was stored in vitro for 1 day, added with NMN, NAC, AMPK inhibitor or NMN+NAC+AMPK inhibitor (NMN 0.5mM, NAC 5mM, AMPKi 4uM) or PBS as a control. They were then labeled with biotin and infused into WT mice. Platelets stored with NMN, NAC, or AMPK inhibitor survived in vivo better than controls, and the combination of the three compounds was significantly superior to individual use (Figure 8C).

### Example 9. Combination of CPT2 up-regulator, CPT1 inhibitor and antioxidant significantly improves platelet survival in immune thrombocytopenia (ITP)

ITP is an autoimmune disease characterized by low platelet counts, leading to potentially fatal bleeding. Previous studies have shown that platelets in patients with autoimmune antibodies in ITP undergo damage and apoptosis, resulting in a shorter lifespan. To evaluate the effects of NMN, NAC or AMPK inhibitor on platelet survival in ITP patients, an ITP mouse model was established by intravenous injection of a low dose of anti-CD42b antibody R300.

The results showed that the platelet survival rate and the recovery rate of platelet counts in the NMN, NAC or AMPK inhibitor group were higher than those in the control group, especially in the NMN+NAC+AMPKi group (1 µmol NMN, 10 µmol NAC, 8 nmol AMPKi) (Figure 8D).

Schematic diagram of the intracellular mechanism involved in CPT2 is shown in Figure 8E.

### Example 10. Combination of CPT2 up-regulator, CPT1 inhibitor, and antioxidant significantly improves the quality of refrigerated platelets

Low temperatures can cause platelet deformation and activation, leading to rapid clearance of platelets after transfusion. Therefore, the widespread use of refrigerated platelets is limited. However, the benefit of refrigerated platelets is the inhibition of bacterial growth that may cause infection.

The inventors also examined the effects of NMN, NAC, and AMPKi on refrigerated platelets. The results showed that the aggregation activity of platelets added with NMN+NAC+AMPKi was significantly higher than that of the control group, and the apoptosis was significantly lower than that of the control group (Figure 9). This indicates that the combination of NMN, NAC, and AMPKi can also prolong the lifespan of refrigerated platelets and improve the quality of refrigerated platelets.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A use of carnitine palmitoyltransferase II or an up-regulator thereof in preparation of a composition, wherein the composition has one or more functions selected from the following group: (1) maintaining or improving the activity of platelets, (2) maintaining or improving storage quality of platelets, (3) reducing platelet apoptosis, (4) prolonging platelet lifespan and/or (5) increasing platelet counts.

2. The use according to claim 1, wherein the up-regulator of carnitine palmitoyltransferase II comprises one or more selected from the following group: (a) a substance capable of reducing acetylation of carnitine palmitoyltransferase II; (b) a substance capable of increasing activity of carnitine palmitoyltransferase II; (c) a substance capable of improving expression, stability or effective time of carnitine palmitoyltransferase II.

3. The use according to claim 2, wherein the up-regulator of carnitine palmitoyltransferase II comprises one or more selected from the following group:
an agent targeting acetylation site of carnitine palmitoyltransferase II to reduce or abolish acetylation of the site; preferably, substance capable of reducing acetylation of carnitine palmitoyltransferase II is a protein acetylation inhibitor; preferably, the protein acetylation inhibitor comprises a down-regulator that reduces acetyltransferase gene or protein expression and/or activity; preferably, the protein acetylation inhibitor is a mitochondrial protein acetylation inhibitor; preferably, the protein acetylation inhibitor is specific to CPT2; preferably, the acetylation site is lysine 79 of carnitine palmitoyltransferase II; preferably, the agent comprises: an agent for site-directed mutagenesis or site-directed modification; preferably, the down-regulator that reduces acetyltransferase gene or protein expression and/or activity comprises down-regulators of gene editing, gene knockout, siRNA, microRNA, or small molecules; preferably, the protein acetylation inhibitor is SIRT3 or an activator thereof;
CPT1 inhibitors; preferably, the CPT1 inhibitors comprise CPT1 inhibitors to itself, upstream AMPK inhibitors, ACC agonists; preferably, the CPT1 inhibitors comprise: Malonyl-CoA, Etomoxir, Perhexiline maleate, Dorsomorphin 2HCl;
protein acetylation inhibitors; preferably comprising: lysine acetyltransferase inhibitors, mitochondrial protein acetylation inhibitors, or activators or expression up-regulators thereof; preferably, the lysine acetyltransferase inhibitors comprise: tip60 inhibitors or p300 inhibitors; preferably, the mitochondrial protein acetylation inhibitors comprise: SIRT3; preferably, activators of SIRT3 comprise: agents that increase concentration of NAD[+] precursors or agents that increase NAMPT enzyme activity; preferably, agents that increase concentration of NAD[+] precursors comprise: nicotinamide mononucleotide, nicotinamide, nicotinamide riboside; preferably, the agents that increase NAMPT enzyme activity comprise: P7C3, SBI-797812;
AMPK inhibitors; preferably comprising: Dorsomorphin 2HCl;
antioxidants; preferably comprising: N-acetylcysteine, Mesna or glutathione;
an expression construct for recombinantly expressing carnitine palmitoyltransferase II;
an up-regulator that promotes the driving ability of carnitine palmitoyltransferase II gene promoter; or
a down-regulator of carnitine palmitoyltransferase II gene-specific microRNA.

4. A method for storing platelets in vitro and/or improving quality of platelets stored in vitro, wherein the method comprises treating steps selected from the following group during platelet storage: (a) decreasing acetylation of carnitine palmitoyltransferase II (CPT2); (b) enhancing activity of carnitine palmitoyltransferase II; (c) increasing expression, stability, or effective time of carnitine palmitoyltransferase II; or (d) adding active carnitine palmitoyltransferase II.

5. The method according to claim 4, wherein the method comprises treating with the up-regulators of carnitine palmitoyltransferase II selected from the following group:
an agent targeting acetylation site of carnitine palmitoyltransferase II to reduce or abolish acetylation of the site; preferably, substance that reduces acetylation of carnitine palmitoyltransferase II is a protein acetylation inhibitor; preferably, the protein acetylation inhibitor comprises a down-regulator that reduces acetyltransferase gene or protein expression and/or activity; preferably, the protein acetylation inhibitor is a mitochondrial protein acetylation inhibitor; preferably, the protein acetylation inhibitor is specific to CPT2; preferably, the acetylation site is lysine 79 of carnitine palmitoyltransferase II; preferably, the agent comprises: an agent for site-directed mutagenesis or site-directed modification; preferably, the down-regulator that reduces acetyltransferase gene or protein expression and/or activity comprises down-regulators of gene editing, gene knockout, siRNA, microRNA, or small molecules; preferably, the protein acetylation inhibitor is SIRT3 or an activator thereof;
CPT1 inhibitors; preferably, the CPT1 inhibitors comprise CPT1 inhibitors to itself, upstream AMPK inhibitors, ACC agonists; preferably, the CPT1 inhibitors comprise: Malonyl-CoA, Etomoxir, Perhexiline maleate, Dorsomorphin 2HCl;
protein acetylation inhibitors; preferably comprising: lysine acetyltransferase inhibitors, mitochondrial protein acetylation inhibitors, or activators or expression up-regulators thereof; preferably, the lysine acetyltransferase inhibitors comprise: tip60 inhibitors or p300 inhibitors; preferably, the mitochondrial protein acetylation inhibitors comprise: SIRT3; preferably, activators of SIRT3 comprise: agents that increase concentration of NAD[+] precursors or agents that increase NAMPT enzyme activity; preferably, agents that increase concentration of NAD[+] precursors comprise: nicotinamide mononucleotide, nicotinamide, nicotinamide riboside; preferably, the agents that increase NAMPT enzyme activity comprise: P7C3, SBI-797812;
AMPK inhibitors; preferably comprising: Dorsomorphin 2HCl;
antioxidants; preferably comprising: N-acetylcysteine, mesna or glutathione;
an expression construct for recombinantly expressing carnitine palmitoyltransferase II;
an up-regulator that promotes the driving ability of carnitine palmitoyltransferase II gene promoter; or
a down-regulator of carnitine palmitoyltransferase II gene-specific microRNA.

6. A composition, wherein the composition has one or more functions selected from the following group: (1) maintaining or improving activity of platelets, (2) maintaining or improving storage quality of platelets, (3) reducing platelet apoptosis, (4) prolonging platelet lifespan and/or (5) increasing platelet counts; the composition comprises an up-regultor of carnitine palmitoyltransferase II, comprising one or more selected from the following group: (a) a substance capable of reducing acetylation of carnitine palmitoyltransferase II; (b) a substance capable of increasing activity of carnitine palmitoyltransferase II; (c) a substance capable of improving expression, stability or effective time of carnitine palmitoyltransferase II; preferably, the up-regultor of carnitine palmitoyltransferase II comprises one or more selected from the following group:
an agent targeting acetylation site of carnitine palmitoyltransferase II to reduce or abolish the acetylation of the site; preferably, substance capable of reducing acetylation of carnitine palmitoyltransferase II is a protein acetylation inhibitor; preferably, the protein acetylation inhibitor comprises a down-regulator that reduces acetyltransferase gene or protein expression and/or activity; preferably, the protein acetylation inhibitor is a mitochondrial protein acetylation inhibitor; preferably, the protein acetylation inhibitor is specific to CPT2; preferably, the acetylation site is lysine 79 of carnitine palmitoyltransferase II; preferably, the agent comprises: an agent for site-directed mutagenesis or site-directed modification; preferably, the down-regulator that reduces acetyltransferase gene or protein expression and/or activity comprises down-regulators of gene editing, gene knockout, siRNA, microRNA, or small molecules; preferably, the protein acetylation inhibitor is SIRT3 or an activator thereof;
CPT1 inhibitors; preferably, the CPT1 inhibitors comprise CPT1 inhibitors to itself, upstream AMPK inhibitors, ACC agonists; preferably, the CPT1 inhibitors comprise: Malonyl-CoA, Etomoxir, Perhexiline maleate, Dorsomorphin 2HCl;
protein acetylation inhibitors; preferably comprising: lysine acetyltransferase inhibitors, mitochondrial protein acetylation inhibitors, or activators or expression up-regulators thereof; preferably, the lysine acetyltransferase inhibitors comprise: tip60 inhibitors or p300 inhibitors; preferably, the mitochondrial protein acetylation inhibitors comprise: SIRT3; preferably, activators of SIRT3 comprise: agents that increase concentration of NAD[+] precursors or agents that increase NAMPT enzyme activity; preferably, agents that increase the concentration of NAD[+] precursors comprise: nicotinamide mononucleotide, nicotinamide, nicotinamide riboside; preferably, the agents that increase NAMPT enzyme activity comprise: P7C3, SBI-797812;
AMPK inhibitors; preferably comprising: Dorsomorphin 2HCl;
antioxidants; preferably comprising: N-acetylcysteine, mesna or glutathione;
an expression construct for recombinantly expressing carnitine palmitoyltransferase II;
an up-regulator that promotes the driving ability of carnitine palmitoyltransferase II gene promoter; or
a down-regulator of carnitine palmitoyltransferase II gene-specific microRNA.

7. The composition according to claim 6, wherein the composition comprises: a protein acetylation inhibitor, an antioxidant and a CPT1 inhibitor;
preferably, the protein acetylation inhibitors is nicotinamide mononucleotide, the antioxidant is N-acetylcysteine, the CPT1 inhibitor is an AMPK inhibitor;
more preferably, the nicotinamide mononucleotide, N-acetylcysteine and AMPK inhibitor are in a molar ratio of (50~500):(500~5000):1; more preferably (80∼300):(800∼3000):1;
more preferably, the nicotinamide mononucleotide added in platelets or substances comprising platelets is in a concentration of 0.1-5000uM; or, the N-acetylcysteine added in platelets or substances comprising platelets is in a concentration of 1-50000uM; or the AMPK inhibitor added in platelets or substances comprising platelets is in a concentration of 0.8-40000nM.

8. A drug kit, wherein it comprises the composition according to claim 6 or 7.

9. A use of the composition according to claim 6 or 7 or the drug kit according to claim 8, for (1) maintaining or improving activity of platelets, (2) maintaining or improving the storage quality of platelets, (3) reducing platelet apoptosis, (4) prolonging platelet lifespan and/or (5) increasing platelet counts.

10. A use of carnitine palmitoyltransferase II for screening drugs, wherein the drug has a function selected from the following group: (1) maintaining or improving activity of platelets, (2) maintaining or improving storage quality of platelets, (3) reducing platelet apoptosis, (4) prolonging platelet lifespan and/or (5) increasing platelet counts.

11. A method for screening drugs able of maintaining or improving activity of platelets, or maintaining or improving storage quality of platelets, wherein the method comprises:
(1) treating an expression system by a candidate substance, wherein the system expressing carnitine palmitoyltransferase II; and
(2) detecting the carnitine palmitoyltransferase II in the system, if the candidate substance has a function selected from the following group, it indicates that the candidate substance is the desired drug;
(i) reducing or abolishing acetylation at the acetylation site of carnitine palmitoyltransferase II; preferably, the acetylation site is lysine 79 of carnitine palmitoyltransferase II;
(ii) improving expressions of carnitine palmitoyltransferase II; or
(iii) improving activities of carnitine palmitoyltransferase II.

12. A genetically engineered platelet or a precursor thereof, wherein there is an exogenous carnitine palmitoyltransferase II or an up-regulator thereof converted in the platelet or the precursor; preferably, the up-regulator of carnitine palmitoyl transferase II comprises one or more selected from the following group:
an agent targeting acetylation site of carnitine palmitoyltransferase II to reduce or abolish the acetylation of the site; preferably, the substance that reduces acetylation of carnitine palmitoyltransferase II is a protein acetylation inhibitor; preferably, the protein acetylation inhibitor comprises a down-regulator that reduces acetyltransferase gene or protein expression and/or activity; preferably, the protein acetylation inhibitor is a mitochondrial protein acetylation inhibitor; preferably, the protein acetylation inhibitor is specific to CPT2; preferably, the acetylation site is lysine 79 of carnitine palmitoyltransferase II; preferably, the agent comprises: an agent for site-directed mutagenesis or site-directed modification; preferably, the down-regulator that reduces acetyltransferase gene or protein expression and/or activity comprises down-regulators of gene editing, gene knockout, siRNA, microRNA, or small molecules; preferably, the protein acetylation inhibitor is SIRT3 or an activator thereof;
protein acetylation inhibitors; preferably comprising: lysine acetyltransferase inhibitors, mitochondrial protein acetylation inhibitors, or expression up-regulators thereof; preferably, the mitochondrial protein acetylation inhibitors comprise: SIRT3;
an expression construct for recombinantly expressing carnitine palmitoyltransferase II;
an up-regulator that promotes the driving ability of carnitine palmitoyltransferase II gene promoter; or
a down-regulator of carnitine palmitoyltransferase II gene-specific microRNA.
